# FASCICULE DE BREVET EUROPEEN

(11) **EP 3 774 950 B1**
(45) Date de publication et mention de la délivrance du brevet: **26.03.2025**
(21) Numéro de dépôt: 19722168.2
(22) Date de dépôt: 04.04.2019
(51) Int. Cl.: C08F 220/58, C11D 3/37, C08F 2/30, A61K 8/06, A61K 8/81

(54) **COMPOSITION AQUEUSE LIQUIDE DÉTERGENTE À USAGE MÉNAGER OU INDUSTRIEL COMPRENANT UN LATEX INVERSE AUTO-INVERSIBLE AVEC LE MONOMÈRE ACRYLAMIDE, COMPRENANT DES ESTERS DE POLYGLYCÉROLS, SON UTILISATION COMME AGENT ÉPAISSISSANT D'UNE FORMULATION DÉTERGENTE OU NETTOYANTE**
WÄSSERIGE FLÜSSIGE REINIGUNGSMITTELZUSAMMENSETZUNG FÜR DEN HAUSHALT ODER DIE INDUSTRIELLE VERWENDUNG, DIE EINEN SELBSTINVERSEN INVERSEN LATEX MIT DEM ACRYLAMID-MONOMER ENTHÄLT, DER POLYGYCEROL-ESTER ENTHÄLT, UND SEINE VERWENDUNG ALS VERDICKUNGSMITTEL IN EINER WASCH- ODER REINIGUNGSMISCHUNG
AQUEOUS LIQUID DETERGENT COMPOSITION FOR HOUSEHOLD OR INDUSTRIAL USE COMPRISING A SELF-INVERTIBLE INVERSE LATEX WITH THE ACRYLAMIDE MONOMER, COMPRISING POLYGLYCEROL ESTERS, AND USE THEREOF AS THICKENING AGENT IN A DETERGENT OR CLEANING FORMULATION

(30) Priorité: 06.04.2018 FR 1853013
(43) Date de publication de la demande: 17.02.2021
(73) Titulaire: Société d'Exploitation de Produits pour les Industries Chimiques SEPPIC, 75321 Paris cedex 07 (FR)
(72) Inventeur: BODOC, Miruna, 81500 Lavaur (FR); DACOSTA, Georges, 81710 SAIX (FR); GUILBOT, Jérôme, 81100 CASTRES (FR); PIERRE, Aurélie, 78160 MARLY LE ROI (FR)
(74) Mandataire: Air Liquide
(86) Numéro de dépôt international: PCT/FR2019/050799
(87) Numéro de publication internationale: WO 2019/193295

(56) Documents cités:
- EP-A1- 1 055 451
- WO-A2-2009/156690
- WO-A2-2009/156691

## Description

L'invention concerne des compositions aqueuses liquides détergentes (F) à usage ménager ou industriel comprenant des latex inverses auto-inversibles comprenant, comme agent inverseur, une composition tensioactive comprenant des esters de polyglycérols et du glycérol et/ou des oligomères du glycérol, l'utilisation desdits latex inverses auto-inversibles comme épaississants utilisés pour préparer des formulations détergentes ou nettoyantes à usage industriel ou ménager, ainsi que ces formulations.

Ces formulations détergentes ou nettoyantes à usage industriel ou ménager soit alcalines soit acides. Les formulations alcalines sont généralement utilisées pour éliminer des surfaces dures, les salissures de matières grasses, alors que les formulations acides le sont, non seulement pour éliminer les salissures, et mais aussi pour détartrer lesdites surfaces. Elles sont particulièrement adaptées au nettoyage et au détartrage d'installations de l'industrie agro-alimentaire, ou au détartrage d'équipements électroménagers, tels que lave-vaisselle et machines à café. Elles sont aussi utilisées pour éliminer des résidus de béton ou de ciment, et pour des opérations de nettoyage de graisses présentes en profondeur sur des surfaces de béton avant toute opération de peinture desdites surfaces de béton. Les formulations détergentes acides ne doivent ne pas provoquer une formation importante de mousse lors de l'opération de nettoyage en présence de la salissure à traiter, elles doivent avoir de bonnes propriétés mouillantes et détergentes. Les formulations détergentes ou nettoyantes à usage industriel ou ménager se présentent sous forme de poudres, de concentrés, de liquides tels que les émulsions, et selon les cas, sont mises en oeuvre directement ou après dilution dans un solvant approprié. Sous forme liquide, telle que les émulsions, elles comportent des modificateurs de rhéologie lors de leur fabrication.

Ces modificateurs de rhéologie sont de préférence des agents épaississants et/ou gélifiants qui ont pour objet d'épaissir la phase aqueuse ou l'émulsion comprenant les tensioactifs détergents, de façon à permettre à l'utilisateur d'en maîtriser l'écoulement, par exemple par l'intermédiaire d'une pompe à circulation, et également à pouvoir suspendre et/ou stabiliser des particules solides. Parmi les agents modificateurs de rhéologie que l'on peut utiliser pour la préparation de formulations détergentes ou nettoyantes à usage industriel ou ménager », on peut citer des polymères synthétiques comme par exemple les polyélectrolytes anioniques, ou cationiques, ou ampholytes, linéaires ou ramifiés, réticulés ou non réticulés, se présentent sous deux formes physiques, la forme poudre et la forme liquide.

La demande internationale publiée sous le numéro WO 2016/020622 A1 décrit l'utilisation, pour la fabrication d'une composition aqueuse liquide détergente à usage ménager ou industriel, d'un polymère branché ou réticulé obtenu par polymérisation d'une solution aqueuse d'un ou plusieurs monomères en émulsion inverse de type eau-dans-huile, avec au moins un des monomères utilisés étant un monomère acrylique et un ou plusieurs des monomères utilisés étant un monomère porteur d'au moins une fonction acide faible, le pourcentage molaire de monomères porteurs d'au moins une fonction acide faible par rapport à l'ensemble des monomères utilisés étant au moins de 30%.

Dans les procédés de préparation de latex inverses auto-inversibles par la mise en oeuvre d'une polymérisation radicalaire en émulsion inverse, les tensioactifs de type huile-dans-eau sont ajoutés à l'issue de l'étape de polymérisation. Leur ajout a pour objet de modifier et de régler la balance hydrophile-lipophile de l'émulsion eau-dans-huile comprenant le polymère (également nommée « latex inverse ») de façon à obtenir un mélange qui, une fois ajouté dans une phase polaire comme par exemple l'eau, changera de sens d'émulsion pour passer de la forme eau-dans-huile vers la forme huile-dans-eau, permettant alors de mettre en contact le polymère précédemment préparé avec la phase polaire à épaissir. Lors d'un tel phénomène physique, le polymère de type polyélectrolyte réticulé et/ou branché se déploie dans ladite phase polaire et forme un réseau tridimensionnel permettant à la phase polaire de gonfler, ce qui se manifeste par une augmentation de la viscosité de cette phase polaire.

Le mélange comprenant le « latex inverse » et le tensioactif de type huile-dans-eau est nommé latex inverse auto-inversible et ledit tensioactif de type huile-dans-eau est nommé « inverseur » ou « agent inverseur ».

Les agents inverseurs couramment utilisés pour la préparation des latex inverses auto-inversibles sont des agents tensioactifs de type huile-dans-eau qui possèdent une valeur de HLB (« Hydrophilic Lipophilic Balance ») suffisamment élevée pour permettre de préparer des émulsions de type huile-dans-eau stables, généralement supérieur à 9 et inférieur à 16. Ils comprennent généralement une partie hydrophile constituée par un enchaînement de motifs d'oxyde d'éthylène et une partie consistant en une chaîne aliphatique hydrocarbonée de nature hydrophobe. Parmi ces agents inverseurs, il y a :
- Les alcools gras éthoxylés, dont la chaîne aliphatique hydrocarbonée comporte de 8 à 14 atomes de carbone et dont le nombre de motifs d'oxyde d'éthylène est compris entre 5 et 40, par exemple l'alcool laurique éthoxylé à 7 moles d'oxyde d'éthylène (nom INCI : Laureth-7), ou l'alcool tridécylique à 6 moles d'oxyde d'éthylène (nom INCI : trideceth-6) ;
- Les esters de sorbitan éthoxylés, dont la chaîne aliphatique hydrocarbonée comporte de 12 à 22 atomes de carbone et dont le nombre de motifs d'oxyde d'éthylène est compris entre 5 et 40, par exemple l'oléate de sorbitan éthoxylé à 20 moles d'oxyde d'éthylène commercialisé sous le nom commercial Montanox^{™}80, ou le laurate de sorbitan éthoxylé à 20 moles d'oxyde d'éthylène commercialisé sous le nom commercial Montanox^{™}20 ;
- Les alkylphénols éthoxylés, par exemple les nonylphénols éthoxylés et les octylphénols éthoxylés ; ou
- Les huiles de ricin éthoxylées, par exemple l'huile de ricin éthoxylée à 40 moles d'oxyde d'éthylène commercialisée sous le nom de marque SIMULSOL^{™}OL 50.

L'évolution des exigences des consommateurs et des dispositions réglementaires amènent les formulateurs de compositions cosmétiques à diminuer la proportion d'ingrédients comportant des motifs d'oxyde d'éthylène dans leurs formulations. Il existe donc un besoin de préparer des latex inverses auto-inversibles exempts de tensioactifs éthoxylés comme agents inverseurs.

Les demandes de brevet français publiées sous les numéros 2 794 034, 2 794 124, 2 808 447, 2 808 446 et 2 810 883 décrivent l'utilisation d'alkyl polyglycosides, dont la chaîne alkyle hydrocarbonée comporte d'un à trente atomes de carbone comme agents inverseurs pour préparer des latex inverses auto-inversibles, comme par exemple des mélanges d'alkyl polyglucosides dont les chaînes alkyles hydrocarbonées sont des chaînes décyle, dodécyle et tétradécyle comme le mélange commercialisé sous le nom de marque SIMULSOL^{™}SL 10, des chaînes dodécyle, tétradécyle et hexadécyle comme le mélange commercialisé sous le nom de marque SIMULSOL^{™}SL 26, des chaînes octyle et décyle comme le mélange commercialisé sous le nom de marque SIMULSOL^{™}SL 8, ou la chaîne undécylènyle comme le mélange commercialisé sous le nom de marque SIMULSOL^{™}SL 11W.

Cependant, la mise en oeuvre de tels composés pour préparer des latex inverses auto-inversibles, doit être réalisée à une température supérieure à leur point de fusion, généralement à 70°C, ce qui pose des problèmes d'augmentation de la viscosité du latex inverse et entraîne une certaine déstabilisation dudit latex inverse auto- inversible préparé. Dans certain cas, elle est réalisée en diluant préalablement lesdits alkyl polyglycosides dans l'eau pour disposer d'une forme liquide et manipulable à température ambiante. Ceci a parfois pour conséquence de diminuer la vitesse d'inversion desdits latex inverses auto-inversibles dans les phases polaires à épaissir, et donc de diminuer la productivité des procédés de préparation des formulations cosmétiques comprenant de tels agents épaississants.

La demande internationale publiée sous le numéro WO 2009/156691 divulgue l'utilisation d'esters de polyglycérol comme agents inverseurs pour préparer des latex inverses auto-inversibles, par exemple des esters de décaglycérol comme le monolaurate de décaglycérol, l'oléate de décaglycérol, le monocaprylate de décaglycérol ou le monomyristate de décaglycérol. Cependant, leur utilisation conduit à des latex inverses auto-inversibles pour lesquels la vitesse d'inversion dans les phases polaires à épaissir est trop lente et même diminue lorsque le latex inverse auto-inversible est stocké après sa préparation pendant plus d'un mois de préparation.

Les inventeurs ont donc cherché à mettre au point un nouveau système tensioactif inverseur de type huile-dans-eau, compatibles avec les normes environnementales en vigueur, en étant notamment exempts de motifs d'oxyde d'alkylène qui permettent de préparer des latex inverses auto-inversibles :
- qui puissent être utilisables facilement et notamment qui puissent être pompés à 25°C, qui aient une viscosité inférieure ou égale à 8.000 mPa.s, de préférence inférieure ou égale à 5.000 mPa.s, viscosité mesurée à 25°C à l'aide d'un viscosimètre Brookfield RVT et du mobile n°3 à la vitesse de 20 tours/minute,
- qui présentent un aspect lisse, exempts de grains ou de grumeaux, et
- qui possèdent de bonnes propriétés d'inversion dans des phases polaires c'est-à-dire induisant une vitesse d'inversion rapide et fiable.

L'invention, telle que définie dans les revendications, a donc pour objet une composition aqueuse liquide détergente (F) à usage ménager ou industriel comprenant comme agent épaississant, pour 100% de sa masse totale entre 0,1% et 10% massique un latex inverse auto-inversible d'un polyélectrolyte anionique réticulé (P) comprenant, pour 100% molaire :
(a₁) - d'une proportion supérieure ou égale à 25% molaire et inférieure ou égale à 80% molaire, d'unités monomériques issues de l'acide 2-méthyl 2-[(1-oxo 2-propènyl) amino] 1-propanesulfonique sous forme acide libre ou partiellement ou totalement salifiée ;
(a₂) - d'une proportion supérieure ou égale à 20% molaire et inférieure ou égale à 75% molaire, d'unités monomériques issues d'au moins un monomère choisi parmi les éléments du groupe constitué par l'acrylamide, le N,N-diméthyl acrylamide ; le méthacrylamide ou le N-isopropyl acrylamide ;
(a₃) - optionnellement d'une proportion supérieure à 0% molaire et inférieure ou égale à 10% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée ;
(a₄) - d'une proportion supérieure à 0% molaire et inférieure ou égale à 1% molaire d'unités monomériques issues d'au moins un monomère de réticulation (AR) diéthylénique ou polyéthylénique;
la somme des dites proportions molaires en unités monomériques selon a₁), a₂) a₃) et a₄) étant égale à 100% molaire;
ledit latex inverse auto-inversible étant une émulsion de type eau-dans-huile (E) comprenant pour 100% de sa masse :
**a)**- de 10% massique à 90% massique, dudit polyélectrolyte anionique réticulé (P) ;
**b)**- De 5% massique à 50% massique, d'une phase grasse constituée d'au moins une huile (H),
**c)**- De 1% massique à 50% massique d'eau,
**d)**- De 0,5% massique à 10% massique d'un système émulsionnant de type eau-dans-huile (S₁), et
**e)**- De 2% massique à 10% massique d'un système émulsionnant de type huile-dans-eau (S₂) ;

la somme des proportions massiques en composés selon a), b), c), d) et e) étant égale à 100% massique ;
ledit système émulsionnant de type huile-dans-eau (S₂) du latex inverse auto-inversible comprenant pour 100% de sa masse :
   **f)** - Une proportion supérieure ou égale à 50% massique et inférieure ou égale à 100% d'une composition (Ce) qui comprend pour 100% de sa masse :
   **e₁)** - De 10% massique à 60% massique d'au moins un composé de formule (I) :

      HO-[CH₂-CH(OH)-CH₂-O]ₙ-H (I)

      dans laquelle n représente un nombre entier supérieur ou égal à un et inférieur ou égal à quinze ;
   **e₂)** - De 40% massique à 90 % massique d'au moins un composé de formule (II) :

      R₁-(C=O)-[O-CH₂-CH(OH)-CH₂]ₚ-OH (II),

      dans laquelle p, différent ou identique à n, représente un nombre entier supérieur ou égal à un et inférieur ou égal à quinze ; et dans laquelle le groupe R₁-(C=O)- représente un radical aliphatique saturé ou insaturé, linéaire ou ramifié, comportant de six à vingt-deux atomes de carbone, et optionnellement
   **e₃)** - Jusqu'à 30% massique d'au moins une composition (C₁₁) représentée par la formule (III) :

      HO-[CH₂-CHOH-CH₂-O-]_{q}-(G)ᵣ-H (III),

      dans laquelle q différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00,
   ladite composition (C₁₁) consistant en un mélange des composés de formules (III₁), (III₂), (III₃), (III₄) et (III₅) :

      HO-[CH₂-CHOH-CH₂-O-]_{q} -O-(G)₁-H (III₁),

      HO-[CH₂-CHOH-CH₂-O-]_{q} -O-(G)₂-H (III₂),

      HO-[CH₂-CHOH-CH₂-O-]_{q} -O-(G)₃-H (III₃),

      HO-[CH₂-CHOH-CH₂-O-]_{q} -O-(G)₄-H (III₄),

      HO-[CH₂-CHOH-CH₂-O-]_{q} -O-(G)₅-H (III₅),

      en des proportions molaires en dits composés de formules (III₁), (III₂), (III₃), (III₄) et (III₅) respectivement égales à a₁, a₂, a₃, a₄ et a₅, telles que la somme (a₁ + a₂ + a₃ + a₄ + a₅) est égale à un, et que la somme (a₁ + 2a₂ + 3a₃ + 4a₄ + 5a₅) est égale à r ;
   la somme des proportions massiques en composés selon e₁), e₂) et e₃) étant égale à 100% massique.

Au sens de la présente invention, par polyélectrolyte anionique réticulé (P), on désigne un polyélectrolyte non linéaire qui se présente à l'état d'un réseau tridimensionnel insoluble dans l'eau, mais gonflable à l'eau et conduisant alors à l'obtention d'un gel chimique.

Au sens de la présente invention, le terme «salifié» indique que la fonction acide présente dans un monomère se trouve sous une forme anionique associée sous forme de sel à un cation, notamment les sels de métaux alcalins, tels que les cations du sodium ou du potassium, ou comme les cations de base azotés tels que le sel d'ammonium, le sel de lysine ou le sel de monoéthanolamine (HOCH₂-CH₂-NH₄⁺). Il s'agit de préférence des sels de sodium ou d'ammonium.

Selon un aspect particulier de la présente invention, ledit latex inverse auto-inversible tel que défini ci-dessus comprend de 20% massique à 90% massique, et plus particulièrement de 30% massique à 90% massique, plus particulièrement de 30% massique à 80% massique, et encore plus particulièrement de 33% massique à 80% massique dudit polyélectrolyte anionique (P) réticulé.

Selon un autre aspect particulier de la présente invention, la proportion molaire en unités monomériques issues de l'acide 2-méthyl 2-[(1-oxo 2-propènyl) amino] 1-propanesulfonique sous forme acide libre ou partiellement ou totalement salifiée présentes dans ledit polyélectrolyte anionique réticulé (P) est supérieure ou égale à 32% molaire et inférieure ou égale à 100% molaire, plus particulièrement supérieure ou égale à 40% molaire et inférieure ou égale à 100% molaire.

Selon un aspect particulier de la présente invention, l'acide 2-méthyl 2-[(1-oxo 2-propènyl) amino] 1-propanesulfonique est sous forme de sel de de sodium ou d'ammonium.

Selon une première alternative de la présente invention, ledit polyélectrolyte anionique réticulé (P), est un copolymère de l'acide 2-méthyl 2-[(1-oxo 2-propènyl) amino] 1-propanesulfonique partiellement ou totalement salifiée, et d'au moins un monomère choisi parmi l'acrylamide, le N,N-diméthyl acrylamide ; le méthacrylamide ou le N-isopropyl acrylamide.

Selon une deuxième alternative de la présente invention, ledit polyélectrolyte anionique réticulé (P), est un terpolymère réticulé de l'acide 2-méthyl 2-[(1-oxo 2-propènyl) amino] 1-propanesulfonique partiellement ou totalement salifiée, d'au moins un monomère choisi parmi l'acrylamide, le N,N-diméthyl acrylamide ; le méthacrylamide ou le N-isopropyl acrylamide, et d'au moins un monomère parmi l'acide acrylique, l'acide méthacrylique, l'acide 2-carboxyéthyl acrylique, l'acide itaconique, l'acide maléique ou l'acide 3-méthyl 3-[(1-oxo 2-propènyl) amino] butanoïque, partiellement salifié ou totalement salifié.

Selon un autre aspect particulier de la présente invention, ledit polyélectrolyte anionique réticulé (P) est issu de la polymérisation, pour 100% molaire :
**(a₁)** - d'une proportion supérieure ou égale à 32% molaire et inférieure à 100% molaire, plus particulièrement supérieure ou égale à 40% molaire et inférieure ou égale à 100% molaire, d'unités monomériques issues d'un monomère possédant une fonction acide fort, partiellement salifiée ou totalement salifiée, plus particulièrement d'un sel de sodium ou un sel d'ammonium de l'acide 2-méthyl 2-[(1-oxo 2-propènyl) amino] 1- propanesulfonique ; et
**(a₂)** - d'une proportion supérieure à 0% molaire et inférieure ou égale à 68% molaire, plus particulièrement supérieur à 0% molaire et inférieure ou égale à 60% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide, partiellement salifiés ou totalement salifiés, et/ou parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), et le vinyl pyrrolidone ; et
**(a₃) -** d'une proportion supérieure à 0% molaire et inférieure ou égale à 1% molaire d'unités monomériques issues d'au moins un monomère de réticulation (AR) diéthylénique ou polyéthylénique ; étant entendu que la somme des proportions molaires des unités monomériques (a₁), (a₂) et (a₃) est égale à 100%.

Par au moins un monomère de réticulation (AR) diéthylènique ou polyéthylénique, on désigne, dans la définition dudit polyélectrolyte anionique réticulé (P), on désigne notamment un monomère choisi le méthylène-bis(acrylamide), le diméthacrylate d'éthylèneglycol, le diacrylate de diéthylèneglycol, le diacrylate d'éthylèneglycol, le diallyl urée, le triallylamine, le triméthylol propanetriacrylate ou le méthylène-bis(acrylamide) ou un mélange de ces composés, l'acide diallyoxyacétique ou un de ses sels comme le diallyloxyacétate de sodium, ou un mélange de ces composés ; et plus particulièrement un monomère choisi parmi le diméthacrylate d'éthylèneglycol, le triallylamine, le triméthylol propanetriacrylate ou le méthylène-bis(acrylamide) ou un mélange ces composés.

Selon un autre aspect particulier, ledit monomère de réticulation (AR) est mis en oeuvre dans une proportion molaire inférieure ou égale à 0,5%, plus particulièrement inférieure ou égale à 0,25% et tout particulièrement inférieure ou égales à 0,1% ; elle est plus particulièrement supérieure ou égales à 0,005% molaire.

Selon un autre aspect particulier de la présente invention, ledit polyélectrolyte anionique réticulé (P) est un copolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium et de l'acrylamide ; un copolymère réticulé par le triallylamine et/ou le méthylène-bis(acrylamide), de l'acide 2-méthyl 2-[(1-oxo 2-propènyl) amino] 1-propanesulfonique (γ) partiellement ou totalement salifié sous forme de sel de sodium, et de l'acrylamide (ε), dans un rapport molaire (γ)/(ε) supérieur ou égal à 30/70 et inférieur ou égal à 90/10 ; un copolymère réticulé par le triallylamine et/ou le méthylène-bis(acrylamide), de l'acide 2-méthyl 2-[(1-oxo 2-propènyl) amino] 1-propanesulfonique (γ) partiellement ou totalement salifié sous forme de sel de sodium, et d'acrylamide, (ε) dans un rapport molaire (γ)/(ε) supérieur ou égal à 40/60 et inférieur ou égal à 90/10 ; un terpolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium, de l'acrylamide et de l'acide acrylique partiellement ou totalement salifiés sous forme de sel de sodium ou de sel d'ammonium ; un terpolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 45% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 10%, ou un terpolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 47% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 8%.

Par huile (H), on désigne dans la définition dudit latex inverse auto-inversible, notamment :
- Les alcanes linéaires comportant de onze à dix-neuf atomes de carbone ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
- Les cyclo-alcanes optionnellement substitués par un ou plusieurs radicaux alkyles linéaires ou ramifies ;
- Les huiles blanches minérales, comme celles commercialisées sous les noms suivants : Marcol^{™}52, Marcol^{™}82, Drakeol^{™}6VR, Eolane^{™}130, Eolane^{™}150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen^{™}L15 ou Emogreen^{™}L19 ;
- Les éthers d'alcool gras de formule (IV) :

   Z₁-O-Z₂ (IV),

   dans laquelle Z₁ et Z₂ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.
- Les mono-esters d'acides gras et d'alcools de formule (V) :

   R'₁-(C=O)-O-R'₂ (V),

   dans laquelle R'₁-(C=O) représente un radical acyle, saturé ou insaturé, linéaire ou ramifié, comportant de huit à vingt-quatre atomes de carbone, et R'2 représente, indépendamment de R'₁, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;
- Les di-esters d'acides gras et de glycérol de formule (VI) et de formule (VII) :

   R'₃-(C=O)-O-CH₂-CH(OH)-CH₂-O-(C=O)-R'₄ (VI)

   R'₅-(C=O)-O-CH₂-CH[O-(C=O)-R'₆]-CH₂-OH (VII),

   formules (VI) (VII) dans lesquelles R'₃-(C=O), R'₄-(C=O), R'₅-(C=O), R'₆-(C=O), identiques ou différents, représentent un groupement acyle, saturé ou insaturé, linéaire ou ramifié, comportant de huit à vingt-quatre atomes de carbone ;
- Les tri-esters d'acides gras et de glycérol de formule (VIII) :

   R'₇-(C=O)-O-CH₂-CH[O-(C=O)-R"₈]-CH₂-O-(C=O)-R"₉ (VIII),

   dans laquelle R'₇-(C=O), R'₈-(C=O) et R'₉-(C=O), identiques ou différents, représentent un groupement acyle, saturé ou insaturé, linéaire ou ramifié, comportant de huit à vingt-quatre atomes de carbone.

Selon un autre aspect particulier de la présente invention, ladite huile (H) est choisie parmi l'undécane, le tridécane, l'isododécane ou l'isohexadécane, les mélanges d'alcanes et d'isoalcanes et de cycloalcanes comme le mélange (M₁) tel que défini précédemment et les mélanges commercialisés sous les noms Emogreen^{™}L15, Emogreen^{™}L19, Emosmart^{™}L15, Emosmart^{™}L19, Emosmart^{™}V21, Isopar^{™}L ou Isopar^{™}M ; les huiles blanches minérales commercialisées sous les noms Marcol^{™}52, Marcol^{™}82, Drakeol^{™}6VR, Eolane^{™}130 ou Eolane^{™}150 ; l'hémisqualane, le squalane, le polyisobutène hydrogéné ou le polydécène hydrogéné ; le dioctyl éther ou le didécyl éther ; le myristate d'isopropyle, le palmitate d'hexyle, le palmitate d'octyle, l'isostéarate d'isostéaryle, l'octanoyl/décanoyl triglycéride, l'hexadécanoyl/octadécanoyl triglycéride, les triglycérides issus de l'huile de colza, de l'huile de tournesol, de l'huile de lin ou de l'huile de palme.

Dans ledit latex inverse auto-inversible objet de la présente invention, le système émulsionnant (S₁) de type eau-dans-huile est constitué soit d'un seul tensioactif émulsionnant soit d'un mélange de tensioactifs émulsionnants, à condition que ledit système émulsionnant (S₁) résultant ait une valeur de HLB suffisamment faible pour induire la formation d'émulsions de type eau-dans-huile.

Comme tensioactif émulsionnant de type eau-dans-huile, il y a par exemple les esters d'anhydro hexitol et d'acides carboxyliques aliphatiques, linéaires ou ramifiés, saturés ou insaturés, comportant de 12 à 22 atomes de carbone éventuellement substitués avec un ou plusieurs groupes hydroxyles, et plus particulièrement les esters d'anhydro hexitol choisis parmi les anhydro-sorbitols et les anhydro-mannitols et d'acides carboxyliques aliphatiques, linéaires ou ramifiés, saturés ou insaturés, comportant de 12 à 22 atomes de carbone éventuellement substitués avec un ou plusieurs groupes hydroxyles.

Selon un autre aspect particulier de la présente invention, ledit système émulsionnant (S₁) de type eau-dans-huile est choisi parmi les éléments du groupe constitué par le laurate de sorbitan, par exemple celui commercialisé sous le nom Montane^{™}20, le palmitate de sorbitan, par exemple celui commercialisé sous le nom Montane^{™}40, le stéarate de sorbitan, par exemple celui commercialisé sous le nom Montane^{™}60, l'oléate de sorbitan, par exemple celui commercialisé sous le nom Montane^{™}80, le sesquioléate de sorbitan, par exemple celui commercialisé sous le nom Montane^{™}85, le trioléate de sorbitan, par exemple celui commercialisé sous le nom Montane^{™}83, l'isolaurate de sorbitan, l'isostéarate de sorbitan, par exemple celui commercialisé sous le nom Montane^{™}70, le laurate de mannitan, l'oléate de mannitan, ou un mélange de ces esters ; les polyesters de poids moléculaire compris entre 1000 et 3000 et issus de la condensation entre un acide poly(isobutényl) succinique ou son anhydride, tels que l'HYPERMER^{™} 2296, ou le mélange commercialisé sous le nom de marque SIMALINE^{™}IE 501 A, les polyhydroxystéarates de polyglycols de formule (IX) : 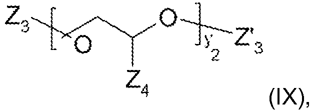 formule (IX) dans laquelle y₂ représente un nombre entier supérieur ou égal à 2 et inférieur ou égal à 50, Z₄ représente l'atome d'hydrogène, le radical méthyle, ou le radical éthyle, Z₃ représente un radical de formule (X) : 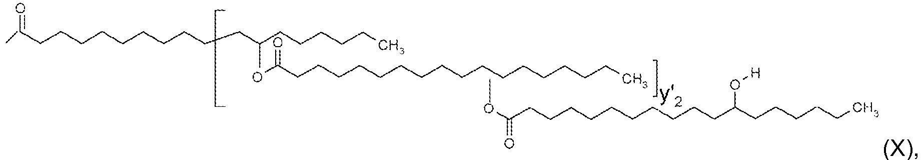 formule (X) dans laquelle y'₂ représente un nombre entier supérieur ou égal à 0 et inférieur ou égal à 10, plus particulièrement supérieur ou égal à 1 et inférieur ou égal à 10 et Z'₃ représente un radical de formule (X) telle que définie ci-dessus, avec Z₃' identique ou différent de Z₃, ou l'atome d'hydrogène.

Comme exemple de tensioactif émulsionnant de type eau-dans-huile de formule (IX) que l'on peut utiliser pour préparer le système émulsionnant (S₁), il y a le PEG-30 dipolyhydroxystéarate commercialisé sous le nom SIMALINE^{™} WO, ou bien les mélanges comprenant le PEG-30 dipolyhydroxystéarate et commercialisés sous les noms SIMALINE^{™}IE 201 A et SIMALINE^{™}IE 201 B, ou encore le mélange comprenant du Triméthylolpropane-30 tripolyhydroxystéarate commercialisé sous le nom SIMALINE^{™}IE 301B.

Dans ledit latex inverse auto-inversible objet de la présente invention, le système émulsionnant (S₂) de type huile-dans-eau est constitué soit de la seule composition (Cₑ), soit d'un mélange de ladite composition (Cₑ) avec un ou plusieurs autres tensioactifs émulsionnants, à condition que ledit système émulsionnant (S₂) résultant ait une valeur de HLB suffisamment élevée pour induire la formation d'émulsions de type huile-dans-eau.

Selon un autre aspect particulier de la présente invention, ledit système émulsionnant (S₂) de type huile-dans-eau comprend pour 100% de sa masse, au moins 75% en masse de ladite composition (Ce) telle que définie précédemment.

Selon un autre aspect particulier de la présente invention, ladite composition (Ce) comprend pour 100% de sa masse :
**e₁)** - De 15% massique à 60% massique, plus particulièrement de 15% massique à 50% massique d'au moins un composé de formule (I) telle que précédemment définie,
**e₂)** - De 40% massique à 85 % massique, plus particulièrement de 50% massique à 85% massique d'au moins un composé de formule (II) telle que définie ci-dessus, et optionnellement
**e₃)** - De 0% massique à 25 % massique d'au moins une composition (C₁₁) représentée par la formule (III) telle que définie précédemment, étant entendu que la somme des proportions massiques des composés selon (e₁), (e₂), (e₃) est égale à 100%.
Selon un autre aspect particulier de la présente invention, dans la formule (I) telle que définie précédemment, n représente un nombre entier supérieur ou égal à un et inférieur ou égal à dix.

Selon un autre aspect particulier de la présente invention, dans la formule (II) telle que définie précédemment, p, identique ou différent à n, représente un nombre entier supérieur ou égal à un et inférieur ou égal à dix.

Selon un autre aspect encore plus particulier, n et p sont identiques et représentent un nombre entier supérieur ou égal à 1 et inférieur ou égal à 10, et encore plus particulièrement supérieur ou égal à 4 et inférieur ou égal à 10.

Selon un autre aspect particulier de la présente invention, dans la formule (II) telle que définie précédemment, le groupe R₁-(C=O)- représente un radical aliphatique saturé ou insaturé, linéaire ou ramifié, comportant de huit à dix-huit atomes de carbone, et encore plus particulièrement le groupe R₁-(C=O)- est choisi parmi les éléments du groupe constitué par les radicaux octanoyle, décanoyle, w-undécylènoyle, dodécanoyle, tétradécanoyle, hexadécanoyle, octadécanoyle, 9-octadécènoyle ou 9,12-octadécadiènoyle.

Selon un autre aspect plus particulier de la présente invention, ledit latex inverse auto-inversible tel que défini précédemment, est **caractérisé en ce que** dans la formule (I) telle que définie précédemment, n représente un nombre entier supérieur ou égal à un et inférieur ou égal à dix, **et en ce que** dans la formule (II) telle que définie précédemment, p, identique ou différent de n, représente un nombre entier supérieur ou égal à un et inférieur ou égal à dix, et le groupe R₁-(C=O)- est choisi parmi les radicaux octanoyle, décanoyle, w-undécylènoyle, dodécanoyle, tétradécanoyle, hexadécanoyle, octadécanoyle, 9-octadécènoyle ou 9,12-octadécadiènoyle.

Selon un autre aspect encore plus particulier de la présente invention, dans lesdites formules (I) et (II) telles que définies précédemment, n est égal à 10, p est égal à 10, et le groupe R₁-(C=O)- est le radical dodécanoyle ; n est égal à 6, p est égal à 10, et le groupe R₁-(C=O)- est le radical dodécanoyle ; n est égal à 6, p est égal à 6, et le groupe R₁-(C=O)- est le radical dodécanoyle ou n est égal à 1, p est égal à 10, et le groupe R₁-(C=O)- est le radical dodécanoyle.

Selon un autre aspect plus particulier de la présente invention, ledit latex inverse auto-inversible tel que défini précédemment est **caractérisé en ce que** dans ledit système émulsionnant de type huile-dans-eau (S₂) ladite composition (Cₑ) telle que définie précédemment, consiste en, pour 100% de sa masse :
**e₁)** - De 10% massique à 60% massique d'au moins un composé de formule (I) telle que définie précédemment et
**e₂)** - De 40% massique à 90 % massique d'au moins un composé de formule (II) telle que définie précédemment.

Par sucre réducteur, on désigne dans la formule (III) telle que définie précédemment, les dérivés saccharidiques qui ne présentent pas dans leurs structures de liaison glycosidique établie entre un carbone anomérique et l'oxygène d'un groupement acétal tels qu'ils sont définis dans l'ouvrage de référence : "Biochemistry", Daniel Voet/Judith G. Voet, p. 250, John Wyley & Sons, 1990. La structure oligomérique (G)ₓ peut se présenter sous toutes formes d'isoméries, qu'il s'agisse d'isomérie optique, d'isomérie géométrique ou d'isomérie de position ; elle peut aussi représenter un mélange d'isomères.

Selon un autre aspect plus particulier de la présente invention, dans la composition (Cₑ) telle que définie précédemment, G représente, dans la formule (III) telle que définie précédemment, le reste d'un sucre réducteur choisi parmi les restes du glucose, du dextrose, du saccharose, du fructose, de l'idose, du gulose, du galactose, du maltose, de l'isomaltose, du maltotriose, du lactose, du cellobiose, du mannose, du ribose, du xylose, de l'arabinose, du lyxose, de l'allose, de l'altrose, du dextrane ou du tallose. Ledit reste G représente encore plus particulièrement dans la formule (III) telle que définie précédemment, un sucre réducteur choisi parmi les restes du glucose, du xylose et de l'arabinose.

Par la formule (III) : HO-CH₂-(CHOH)_{q}-CH₂-O-(G)ᵣ-H, représentant la composition (C₁₁), on signifie que cette composition (C₁₁) consiste essentiellement en un mélange de composés représentés par les formules (III₁), (III₂), (III₃), (III₄) et (III.₅) :

HO-CH₂-(CHOH)_{q}-CH₂-O-(G)₁-H (III₁),

HO-CH₂-(CHOH)_{q}-CH₂-O-(G)₂-H (III₂),

HO-CH₂-(CHOH)_{q}-CH₂-O-(G)₃-H (III₃),

HO-CH₂-(CHOH)_{q}-CH₂-O-(G)₄-H (III₄),

HO-CH₂-(CHOH)_{q}-CH₂-O-(G)₅-H (III₅),

dans les proportions molaires respectives a₁, a₂, a₃, a₄ et a₅, telles que la somme a₁ + a₂ + a₃ + a₄ + a₅ est égale à 1 et que la somme a₁ + 2a₂ + 3a₃ + 4a₄ + 5a₅ est égale à r.

Par essentiellement, on indique dans la définition qui précède, que la présence d'un ou de plusieurs composés de formule (III_{w}) avec w supérieur à 5 n'est pas exclue au sein de la composition (C₁₁), mais que si présence il y a, elle l'est en des proportions minimes qui n'entraînent aucune modification substantielle des propriétés de ladite composition (C₁₁).

Dans la formule (III) telle que définie ci-dessus, le groupe HO-CH₂-(CHOH)_{q}-CH₂-O-est lié à (G)ᵣ par le carbone anomérique du reste saccharide, de manière à former une fonction acétal.

Selon un aspect plus particulier de la présente invention, dans la formule (III) représentant la composition (C₁₁) telle que définie précédemment, r représente un nombre décimal supérieur ou égal à 1,05 et inférieur ou égal à 3, plus particulièrement supérieur ou égal à 1,15 et inférieur ou égal à 2,5.

Selon un aspect plus particulier de la présente invention, ledit latex inverse auto-inversible tel que défini précédemment est **caractérisé en ce que** dans la formule (III) telle que définie précédemment, q est égal à un, G représente le reste du glucose et r représente un nombre décimal supérieur ou égal à 1,05 et inférieur ou égal à 2,5.

Selon un autre aspect particulier de la présente invention, ledit latex inverse auto-inversible tel que défini précédemment est **caractérisé en ce que** dans ledit système émulsionnant de type huile-dans-eau (S₂), ladite composition (Cₑ) telle que définie précédemment consiste en, pour 100% de sa masse :
**e₁)** - De 5% massique à 15% massique d'au moins un composé de formule (I) telle que définie précédemment,
**e₂)** - De 60% massique à 80 % massique d'au moins un composé de formule (II) telle que définie précédemment, et
**e₃)** - De 5% à 15% massique d'au moins une composition (C₁₁) représentée par la formule (III) telle que définie précédemment.

Selon un aspect tout particulier ledit latex inverse tel que défini précédemment est **caractérisé en ce que** ledit système émulsionnant de type huile-dans-eau (S₂) est ladite composition (Cₑ) telle que définie précédemment.

Selon un aspect tout particulier ledit latex inverse auto-inversible tel que défini précédemment, est **caractérisé en ce que** ledit système émulsionnant de type huile-dans-eau (S₂) est ladite composition (Cₑ)

Selon un aspect encore plus particulier de la présente invention, dans la composition (Ce) telle que définie précédemment :
- n représente un nombre entier supérieur ou égal à un et inférieur ou égal à dix dans la formule (I) telle que définie précédemment, et
- p, identique ou différent de n, représente un nombre entier supérieur ou égal à un et inférieur ou égal à dix dans la formule (II) telle que définie précédemment,
- le groupe R₁-(C=O)- est choisi parmi les éléments du groupe constitué par les radicaux octanoyle, décanoyle, w-undécylènoyle, dodécanoyle, tétradécanoyle, hexadécanoyle, octadécanoyle, 9-octadécènoyle ou 9,12-octadécadiènoyle, et
- q est égal à un, G représente le reste du glucose et r représente un nombre décimal supérieur ou égal à 1,05 et inférieur ou égal à 2,5, dans la formule (III) telle que définie précédemment.

Ledit latex inverse auto-inversible objet de la présente invention est préparé par la mise en oeuvre d'un procédé dit de « polymérisation en émulsion inverse », bien connu de l'homme du métier, et qui comprend les étapes suivantes :
- Une étape a) de préparation d'une phase aqueuse comprenant de l'eau, les monomères hydrosolubles et optionnellement le monomère de réticulation (AR), ainsi que des additifs couramment utilisés comme par exemple des agents séquestrants comme l'éthylènediaminetétracétique (EDTA) sous sa forme sodique, ou le sel de penta-sodium de l'acide penta-acétique du diéthylènetétramine (commercialisé sous le nom de marque Versenex^{™}80) ;
- Une étape b) de mélange de la phase huileuse (H) avec le système émulsionnant de type eau-dans-huile (S₁) ;
- Une étape c) de mélange de la phase aqueuse et de la phase huileuse, préparées lors des précédentes étapes, et d'émulsification à l'aide d'un mobile de type rotor-stator ;
- Une étape d) d'inertage à l'azote ;
- Une étape e) d'amorçage de la réaction de polymérisation par introduction dans l'émulsion formée en c), d'un initiateur de radicaux libres et d'éventuellement un co-initiateur ; puis on la laisse se dérouler,
- Une étape f) d'introduction du système émulsionnant (S₂) de type huile-dans-eau tel que défini précédemment à une température inférieure ou égale à 50°C.

Selon un aspect particulier du procédé tel que défini précédemment, la réaction de polymérisation de l'étape e) est amorcée par un couple oxydo-réducteur générateur d'ions hydrogénosulfite (HSO₃⁻), tel que le couple hydroperoxyde de cumène -métabisulfite de sodium (Na₂S₂O₅) ou le couple hydroperoxyde de cumène-chlorure de thionyle (SOCl₂) à une température inférieure ou égale à 10°C, si désiré accompagné d'un agent co-initiateur de polymérisation tel que par exemple l'azo-bis(isobutyronitrile) puis conduite soit de manière quasi adiabatique jusqu'à une température supérieure ou égale à 50° C, soit en contrôlant la température.

Selon un autre aspect particulier du procédé tel que défini précédemment, le milieu réactionnel issu de l'étape e), est concentré par distillation, avant la mise en oeuvre de l'étape f).

Selon un autre aspect particulier du procédé tel que défini précédemment, le milieu réactionnel issu de l'étape e) ou de l'étape f) subit une étape de séchage par atomisation dans une installation adaptée.

Selon un autre aspect particulier du procédé tel que défini précédemment, la phase aqueuse préparée à l'étape a) peut comprendre des agents réducteurs de chaîne, destinés à réduire la longueur des chaînes polymériques formées et à augmenter le taux de branchement sur le polymère, de façon à modifier les propriétés rhéologiques.

Parmi les agents réducteurs de chaîne adaptés au procédé tel que défini précédemment, il y a le méthanol, l'isopropanol, le butylène glycol, le 2-mercapto éthanol, l'acide thioglycolique, l'acide formique ou ses sels.

L'invention a aussi pour objet l'utilisation dudit latex inverse auto-inversible tel que défini précédemment, comme agent épaississant et/ou émulsionnant et/ou stabilisant d'une composition aqueuse liquide détergente à usage ménager ou industriel.

Par « compositions aqueuses liquides détergentes à usage ménager ou industriel », on entend au sens de la présente invention des compositions liquides à 20°C, conçues et utilisées pour le nettoyage de différents types de surfaces, comme par exemple les fibres textiles, les surfaces dures de différentes natures comme par exemple le verre, la céramique, les carrelages, le bois, le métal, les matériaux composites. Ces « compositions aqueuses liquides détergentes à usage ménager ou industriel » trouvent leurs applications pour nettoyer lesdites surface de salissures, comme par exemple le nettoyage de la vaisselle manuellement ou par l'intermédiaire d'une machine lave-vaisselle, des bouteilles, du linge manuellement ou par l'intermédiaire d'une machine lave-linge, des sols, des surfaces métalliques souillées par des graisses, les vitres, les toilettes, les cuves de stockage.

Parmi ces compositions aqueuses liquides détergentes à usage ménager ou industriel destinées à la détergence de surfaces dures pour des applications ménagères ou industrielles, on peut distinguer les compositions nettoyantes aqueuses alcalines et les compositions nettoyantes aqueuses acides. De telles compositions aqueuses liquides détergentes à usage ménager ou industriel peuvent se présenter sous la forme d'une solution, d'un gel, d'une émulsion de type huile-dans-eau ou de type eau-dans-huile, sous la forme d'une dispersion.

Selon un aspect particulier, ladite utilisation consiste à épaissir des phases polaires comme par exemple les phases aqueuses, alcooliques ou hydro-alcooliques ou les phases polaires comprenant des polyols tels que le glycérol.

Selon un autre aspect particulier, ladite utilisation consiste à stabiliser une émulsion de type huile-dans-eau, ou de type eau-dans-huile, en conférant un aspect homogène à ladite émulsion pendant le stockage dans différentes conditions, et plus particulièrement à 25°C pendant une durée au moins égale à un mois, et plus particulièrement à 4°C pendant une durée au moins égale à un mois, et plus particulièrement à 45°C pendant une durée au moins égale à un mois.

Selon un autre aspect particulier, ladite utilisation consiste à stabiliser des particules solides dans des compositions aqueuses liquides détergentes à usage ménager ou industriel.

Ces particules solides à suspendre peuvent revêtir différentes géométries, régulières ou irrégulières, et se présenter sous forme de perles, de billes, de tiges, de paillettes, de lamelles ou de polyèdres. Ces particules solides se caractérisent par un diamètre moyen apparent compris entre un micromètre et cinq millimètres, plus particulièrement entre dix micromètres et un millimètre.

Parmi les particules solides qui peuvent être mises en suspension et stabilisées par le latex inverse auto-inversible telle que définie précédemment dans des compositions aqueuses liquides détergentes à usage ménager ou industriel, il y a les micas, l'oxyde de fer, l'oxyde de titane, l'oxyde de zinc, l'oxyde d'aluminium, le talc, la silice, le kaolin, les argiles, le nitrure de bore, le carbonate de calcium, le carbonate de magnésium, l'hydrogénocarbonate de magnésium, les pigments colorés inorganiques, les polyamides comme le nylon-6, les polyéthylènes, les polypropylènes, les polystyrènes, les polyesters, les polymères acryliques ou méthacryliques comme les polyméthylméthacrylates, le polytétrafluoroéthylène, les cires cristallines ou microcristallines, des sphères poreuses, le sulphide de sélénium, le pyrithione de zinc, les amidons, les alginates, les fibres de végétaux, les particules de Loofah, les particules d'éponges.

L'invention a également pour objet une composition aqueuse liquide détergente (F) à usage ménager ou industriel, **caractérisée en ce qu**'elle comprend comme agent épaississant, pour 100% de sa masse totale entre 0,1% et 10% massique dudit latex inverse auto-inversible, tel que définie précédemment.

Ladite une composition aqueuse liquide détergente (F), objet de la présente invention se présente notamment sous la forme d'une solution aqueuse, d'une émulsion ou d'une microémulsion à phase continue aqueuse, d'une émulsion ou d'une microémulsion à phase continue huileuse, d'un gel aqueux, d'une mousse, ou encore sous la forme d'un aérosol. Elle peut être appliquée directement par trempage, par aspersion ou par vaporisation sur la surface à nettoyer ou bien par l'intermédiaire de tout type de support destiné à être mis en contact avec la surface dure à nettoyer (papier, lingette, textile).

De façon générale ladite composition aqueuse liquide détergente (F) objet de la présente invention, comporte également des ingrédients habituellement mis en oeuvre dans le domaine du nettoyage de surfaces dures ou de fibres textiles, comme les tensioactifs non-ioniques, cationiques ou amphotères, des polymères cationiques ou non-ioniques, des agents épaississants, des enzymes, des agents de blanchiment, des agents anticorrosion, des solvants, des agents acides, des agents alcalins, des agents anticalcaire, des agents conservateurs, des parfums, des colorants, des agents répulsifs, des agents oxydants, des adjuvants de détergence, des agents antisalissure, les agents anti-redéposition.

Parmi les acides minéraux particulièrement choisis comme agents acides dans ladite composition aqueuse liquide détergente (F), on peut citer l'acide chlorhydrique, l'acide nitrique, l'acide phosphorique, l'acide sulfurique, l'acide hypophosphoreux, l'acide phosphoreux, l'acide hypochloreux, l'acide perchlorique, l'acide carbonique, l'acide borique, l'acide manganique, l'acide permanganique, l'acide chromique, l'acide périodique, l'acide iodique, l'acide hypoiodeux, l'acide bromohydrique, l'acide iodhydrique, l'acide fluorhydrique.

Parmi les acides organiques particulièrement choisis comme agents acides dans ladite composition aqueuse liquide détergente (F), on peut citer l'acide formique, l'acide acétique, l'acide propionique, l'acide benzoïque, l'acide salicylique, l'acide oxalique, l'acide malonique, l'acide succinique, l'acide glutarique, l'acide adipique, l'acide glycolique, l'acide lactique, l'acide malique, l'acide maléique, l'acide tartrique, l'acide citrique, l'acide sorbique, l'acide sulfamique, l'acide dihydroacétique, l'acide diméthylsulfamique, l'acide fumarique, l'acide glutamique, l'acide isopropyl sulfamique, l'acide valérique, l'acide benzène sulfonique, l'acide xylène sulfonique, l'acide 2-éthyl-hexanoïque, l'acide caprique, l'acide caproïque, l'acide crésylique, l'acide dodécylbenzène sulfonique, l'acide peracétique, l'acide monochloroacétique, l'acide gluconique.

Parmi les agents alcalins associés au dit latex inverse auto-inversible tel que défini précédemment dans ladite composition aqueuse liquide détergente (F), on peut citer les éléments du groupe constitué par les hydroxydes de métaux alcalins ou alcalino-terreux comme par exemple l'hydroxyde de sodium, l'hydroxyde de potassium, l'hydroxyde de baryum et l'hydroxyde de calcium.

Parmi les agents anti-calcaires associés au dit latex inverse auto-inversible tel que défini précédemment dans ladite composition aqueuse liquide détergente (F), on peut citer les éléments du groupe constitué par les agents séquestrants, comme par exemple le tripolyphospate de sodium (TPP), l'éthylènediaminetétracétate (EDTA), le tetraacetyléthylènediamine (TAED), le méthyl glycine diacétate (MGDA), le nitrolotriacétate de sodium (Na3NTA), les gluconates de sodium ou de potassium, les érythorbates de sodium ou de potassium, les polycarboxylates de sodium ou de potassium, et le citrate de sodium, par les agents échangeurs d'ions comme par exemple les zéolithes ou aluminosilicates de sodium, ou les silicates de sodium lamellaires, les agents précipitants comme par exemple le carbonate de calcium et le métasilicate de sodium.

Les agents séquestrants, et plus particulièrement les agents séquestrants décrits ci-dessus, ont pour effet de complexer les ions calcium et magnésium pour former des complexes hydrosolubles ensuite éliminés pendant le rinçage. Les agents échangeurs d'ions, et plus particulièrement les agents échangeurs d'ions décrits ci-dessus, ont pour effet d'échanger leurs ions sodium avec des ions calcium et magnésium. Les agents précipitants, et plus particulièrement les agents séquestrants décrits ci-dessus, ont pour effet d'éliminer les ions responsables de la dureté de l'eau en formant des composés de calcium insolubles, éliminé par la suite avec les salissures sur les surfaces nettoyées.

Selon un aspect plus particulier, dans ladite composition aqueuse liquide détergente (F), l'agent anticalcaire est choisi parmi les éléments du groupe constitué du métasilicate de sodium, du tripolyphospate de sodium (TPP), de l'éthylènediaminetétracétate (EDTA), du tetraacetyléthylènediamine (TAED), du méthyl glycine diacétate (MGDA), du nitrolotriacétate de sodium (Na3NTA), du gluconate de sodium, du citrate de sodium et du carbonate de calcium.

Parmi les tensioactifs non-ioniques que l'on peut associer au dit latex inverse auto-inversible tel que défini précédemment dans ladite composition aqueuse liquide détergente (F), on peut citer :
- Des copolymères blocs d'oxyde d'éthylène et d'oxyde de propylène, et tout particulièrement les copolymères blocs d'oxyde d'éthylène et d'oxyde de propylène commercialisés sous le nom de marque PLURONIC^{™} par la société BASF, comme par exemple le PLURONIC^{™}PE 6100 et le PLURONIC^{™}PE 6200 ,
- Des tensioactifs non ioniques démoussants de formule (A₁) :

   R₁-X-[(CH₂-CH(CH₃)-O)_{u'}-(CH₂-CH₂-O)_{v'}-Y]_{w'} (A₁)

   dans laquelle R₁ représente un radical aliphatique hydrocarboné, saturé ou insaturé, linéaire ou ramifié, comportant de 6 à 18 atomes de carbone, X représente un atome d'azote ou un atome d'oxygène, u' et v', identiques ou différents, représentent chacun un nombre entier compris entre 1 et 50, w' est soit égal à 1 si X représente un atome d'oxygène, soit égal à 1 ou à 2 si X représente un atome d'azote, et Y représente un groupe fonctionnel bloquant choisi parmi les éléments du groupe constitué par les radicaux alkyles linéaires comportant de 4 à 8 atomes de carbone, comme le radical butyle, le radical benzyle, un groupe oxyde de butylène.

Parmi les tensioactifs non ioniques démoussants de formule (A₁), on peut citer les produits commercialisés sous le nom de marque TERGITOL^{™} par la société DOW CHEMICAL comme par exemple le TERGITOL^{™} L61E et le TERGITOL^{™} L64E
- Des tensioactifs non ioniques peu moussant de formule (A₂) :

   R₈-O-(S')_{q'}-H (A₂)

   dans laquelle S'représente le reste d'un sucre réducteur choisis parmi les éléments du groupe constitué par le glucose, le xylose et l'arabinose, R₈ représente un radical hydrocarboné saturé, linéaire ou ramifié, comportant de 6 à 10 atomes de carbone et q' représente un nombre décimal supérieur ou égal à 1,05 et inférieur ou égal à 5.

Comme exemple de tensioactifs non ioniques peu moussants de formule (A₂) optionnellement présents dans ladite composition aqueuse liquide détergente (F), on peut citer les hexylpolyglucosides, les 2-éthyl hexyl polyglucosides, les n-heptyl polyglucosides ou les n-octyl polyglucosides.

Parmi les tensioactifs amphotères moussants et/ou détergents que l'on peut associer au dit latex inverse auto-inversible tel que défini précédemment dans ladite composition aqueuse liquide détergente (F), on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates, les amphopropionates, le β alanine, N-(2-carboxyethyl)-N(2-ethylhexyl) de sodium commercialisé sous le nom de marque TOMAMINE^{®} 30 AMPHOTERIC 400 SURFACTANT.

Parmi les tensioactifs amphotères moussants et/ou détergents que l'on peut associer au dit latex inverse auto-inversible tel que défini précédemment dans ladite composition aqueuse liquide détergente (F), il y a les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates, le β alanine, N-(2-carboxyethyl)-N(2-ethylhexyl) de sodium commercialisé sous le nom de marque TOMAMINE^{®} 30 AMPHOTERIC 400 SURFACTANT.

Parmi les tensioactifs non ioniques que l'on peut associer au dit latex inverse auto-inversible tel que défini précédemment dans ladite composition aqueuse liquide détergente (F), on peut citer les monoglycérides alcoxylés, les diglycérides alcoxylés, les hydrocarbures terpéniques alcoxylés tels que les α- ou β-pinènes éthoxylés et/ou propoxylés, contenant de 1 à 30 motifs oxyéthylène et/ou oxypropylène, les produits résultant de la condensation de l'oxyde d'éthylène ou de l'oxyde de propylène avec l'éthylènediamine, tels les TETRONIC^{™} commercialisés par BASF, les acides gras éthoxylés et/ou propoxylés en C8-C18 contenant de 5 à 25 moles d'oxyde d'éthylène et/ou de propylène, les amides gras éthoxylés contenant de 5 à 30 moles d'oxyde d'éthylène, les aminés éthoxylées contenant de 5 à 30 moles d'oxyde d'éthylène, les amidoamines alcoxylées contenant de 1 à 50, de préférence de I à 25, tout particulièrement de 2 à 20 moles d'oxyde d'éthylène et/ou de propylène.

Parmi les agents épaississants et/ou gélifiants que l'on peut associer au dit latex inverse auto-inversible tel que défini précédemment dans ladite composition aqueuse liquide détergente (F), on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), 25 de la gomme de fenugrec (DS = 1 ).

Parmi les agents épaississants et/ou gélifiants que l'on peut associer au dit latex inverse auto-inversible tel que défini précédemment dans ladite composition aqueuse liquide détergente (F), on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes. Parmi les agents épaississants et/ou gélifiants que l'on peut associer au dit latex inverse auto-inversible tel que défini précédemment dans ladite composition aqueuse liquide détergente (F), on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Parmi les agents épaississants et/ou gélifiants que l'on peut associer au dit latex inverse auto-inversible tel que défini précédemment dans ladite composition aqueuse liquide détergente (F), on peut citer les agents épaississants inorganiques comme par exemple les argiles, l'hectorite, la saponite, la sauconite, la vermiculite ou la silice colloïdale.
Les agents épaississants présents dans la composition (F) objet de la présente invention sont utilisés dans des quantités comprises entre 0,1% et 10% massique. Parmi les agents abrasifs que l'on peut associer au dit latex inverse auto-inversible tel que défini précédemment dans ladite composition aqueuse liquide détergente (F), on peut citer des matériaux d'origine naturelle comme par exemple des copeaux de bois ou de noyaux, des matériaux abrasifs inorganiques tels que des oxydes, des quartzs, des terres diatomées, des dioxydes de silice colloïdales, des matériaux abrasifs organiques tels que des polyoléfines comme les polyéthylènes et les polypropylènes, des polystyrènes, des résines d'acétonitrile-butadiène-styrène, des mélamines, des résines phénoliques, des résines époxy, des résines polyuréthanes.

Les agents abrasifs présents dans la composition (F) objet de la présente invention sont utilisés dans des quantités comprises entre 5,0% et 30% massique.

Parmi les solvants que l'on peut associer au dit latex inverse auto-inversible tel que défini précédemment dans ladite composition aqueuse liquide détergente (F), on peut citer l'alcool isopropylique, l'alcool benzylique, le 1,3-propanediol, les solvants chlorés, l'acétone, le méthyl éthyl éther, le méthyl isobutyl éther, l'acétate de butyle, l'acétate d'éthyle, l'acétate d'isopropyle, l'acétate d'isobutyle, les solvants aromatiques, les isoparaffines, l'isododécane, le lactate d'éthyle, le lactate de butyle, les solvants terpéniques, les esters méthyliques de colza, les esters méthyliques de tournesol, le propylèneglycol n-méthyl éther, le dipropylène glycol n-méthyl éther, le tripropylène glycol n-méthyl éther, le propylène glycol n-butyl éther, le dipropylène glycol n-butyl éther, le tripropylène glycol n-butyl éther, le propylène glycol n-propyl éther, le dipropylène glycol n-propyl éther, le propylène glycol mono méthyl éther acétate, le propylène glycol di acétate, le propylène glycol phényl éther, l'éthylène glycol phényl éther, le dipropylène glycol diméthyl éther.

Comme exemples de solvants présents dans la composition (F) objet de la présente invention, on peut citer plus particulièrement les éléments du groupe constitué par le propylène glycol n-méthyl éther, le dipropylène glycol n-méthyl éther, le tripropylène glycol n-méthyl éther, le propylène glycol n-butyl éther, le dipropylène glycol n-butyl éther, le tripropylène glycol n-butyl éther, le propylène glycol n-propyl éther, le dipropylène glycol n-propyl éther, le propylène glycol phényl éther, l'éthylène glycol phényl éther, le dipropylène glycol diméthyl éther, les esters méthyliques de colza, les esters méthyliques de tournesol.

Parmi les enzymes que l'on peut associer au dit latex inverse auto-inversible tel que défini précédemment dans ladite composition aqueuse liquide détergente (F), on peut citer les protéases, les amylases, les lipases, les cellulases et les peroxydases.
Les enzymes présentes dans la composition (F) objet de la présente invention sont utilisés dans des quantités comprises entre 0,005 % et 0,5 % massique.

Selon un autre aspect, l'invention a pour objet l'utilisation de ladite composition aqueuse liquide détergente (F) telle que définie précédemment, pour nettoyer les surfaces dures.

L'expression « pour nettoyer les surfaces dures » désigne toute action destinée à permettre l'élimination de salissures présentes sur des surfaces constituées de matériaux divers.

Les surfaces à nettoyer peuvent être des surfaces dures ou des surfaces textiles. Par surfaces dures, on désigne par exemple les sols, les murs, les carreaux de fenêtres, les carrelages, les appareils électro-ménagers, la vaisselle, les plans de travail, la robinetterie, les éviers, des cuves de stockage de produits chimiques, alimentaires ou agricoles, les véhicules (automobiles, moto, camions, ...). Les matériaux constituant ces surfaces dures sont par exemple le verre (sodocalcique, fluorocalcique, borosilicate, cristal), la porcelaine, la faïence, la céramique, les plastiques polycarbonates, polypropylènes, l'acier inoxydable, l'argent, le cuivre, l'aluminium, le bois, les résines synthétiques, la vitrocéramique, le linoleum, et peuvent être revêtus de peintures, de vernis. Comme exemple de salissures présentes sur ces surfaces dures et à éliminer par nettoyage, on peut citer par exemple des résidus alimentaires, des graisses, des hydrocarbures lourds et légers, des résidus brûlés, de la poussière, de la boue, des traces de doigts, des résidus de savon, des germes.

Selon un autre aspect, l'invention a pour objet un procédé de nettoyage d'une surface dure, caractérisé en ce qu'il comprend au moins une étape a"1) d'application de ladite composition aqueuse liquide détergente (F) telle que définie précédemment, suivie d'au moins une étape b"1) de rinçage de ladite surface dure.

Dans l'étape a"1) du procédé tel que défini ci-dessus, ladite composition aqueuse liquide détergente (F) est appliquée sur la surface comprenant les salissures à nettoyer par tout moyen, par exemple en plein bain, par aspersion ou par application par l'intermédiaire d'un support constitué de fibres textiles synthétiques ou naturelles, tissées ou non tissées, ou de papier, préalablement imprégné de celle-ci.

Dans l'étape b"1) du procédé tel que défini ci-dessus, le rinçage de la surface dure sur laquelle a été appliquée la composition (F) à usage ménager ou industriel lors de l'étape a"1) est réalisé en plein bain ou par aspersion d'eau. L'étape b"1) du procédé de nettoyage objet de l'invention, peut être réalisée à température ambiante ou à une température comprise entre 30°C et 80°C, plus particulièrement à une température comprise entre 30°C et 65°C.

Les exemples suivants illustrent l'invention, sans toutefois la limiter.

### I- Préparation de compositions tensioactives selon l'invention et comparatives

### I_{A}- Préparation de la composition (EM₂) à base de laurate de décaglycérol (EM₁) et d'hexaglycérol

On introduit dans un réacteur en verre à double enveloppe, dans laquelle circule un fluide caloporteur, et muni d'une agitation mécanique efficace 71,5 grammes de monolaurate de décaglycérol commercialisé sous le nom de marque DECAGLYN 1-L (ci-après désignée par le terme « Composition (EM₁) »), et 28,5 grammes de polyglycérol-6 (commercialisé sous le nom de marque Polyglycerol6^{™} par la société SPIGA), à une température de 35°C sous une agitation mécanique de type ancre à une vitesse de 80 tours/minute. Après mélange dans de telles conditions pendant 30 minutes, le mélange est vidangé pour obtenir la composition **(EM₂).**

### I_{B}- Préparation de la composition (EM₃) à base de laurate de décaglycérol (EM₁) et de décaglycérol

On introduit dans un réacteur en verre à double enveloppe, dans laquelle circule un fluide caloporteur, et muni d'une agitation mécanique efficace 71,5 grammes de monolaurate de décaglycérol commercialisé sous le nom de marque DECAGLYN 1-L (ci-après désignée par le terme « Composition **(EM₁)** »), et 28,5 grammes de polyglycérol-10 (commercialisé sous le nom de marque Polyglycerin 10^{™}), à une température de 35°C sous une agitation mécanique de type ancre à une vitesse de 80 tours/minute. Après mélange dans de telles conditions pendant 30 minutes, le mélange est vidangé pour obtenir la composition **(EM₃).**

Les caractéristiques analytiques des compositions (EM₁), (EM₂) et (EM₃) sont consignées dans le tableau 1 ci-dessous.

**Tableau 1**

| | Composition émulsionnante | | |
|---|---|---|---|
| | (EM₁) | (EM₂) | (EM₃) |
| Proportions en constituants (% massique) | | | |
| Monolaurate de décaglycérol | 100% | 71,5% | 71,5% |
| Proportion massique d'hexaglycérol | 0% | 28,5 | 0% |
| Proportion massique de décaglycérol | 0% | 0% | 28,5 |

### II- Préparation et évaluation de latex inverses auto-inversibles d'un terpolymère réticulé du sel de sodium de l'acide 2-méthyl-[(1-oxo-2-propènyl) amino] 1-propanesulfonique de l'acrylamide et de l'acide acrylique partiellement salifié et d'un latex inverse auto-inversible d'un copolymère réticulé du sel de sodium de l'acide 2-méthyl-[(1-oxo-2-propènyl) amino] 1-propanesulfonique et de l'acrylamide.

**II-** a) - On prépare une phase aqueuse en versant successivement dans un bécher et sous agitation 277 grammes d'une solution commerciale à 50% massique d'acrylamide, 375 grammes d'une solution aqueuse à 55% de sel de sodium d'acide 2-méthyl-[(1-oxo-2-propenyl) amino] 1-propanesulfonique, 10, 8 grammes d'acide acrylique glacial, 0,058 gramme de méthylène bis(acrylamide), 0,45 gramme d'une solution aqueuse commerciale à 40% en masse de diéthylènetriamine penta-acétate de sodium, et 5,3 grammes d'une solution aqueuse à 48% en masse d'hydroxyde de sodium pour ajuster le pH de cette phase aqueuse à 5,3.

On prépare indépendamment une phase organique en mélangeant 130 grammes de polyisobutène, 90 grammes d'Isopar^{™}H, 30 grammes de Marcol^{™} 52, 17,0 grammes de Montane^{™} 70, 5 grammes de Simaline^{™}IE 200 et 0,2 gramme d'azo bis(isobutyronitryle) (AIBN).

La phase aqueuse préparée est ensuite ajoutée progressivement sur la phase huileuse puis dispersée à l'aide d'un rotor stator type Ultra Turrax commercialisé par la société Ika.

L'émulsion obtenue est transférée dans un réacteur pour être soumise à un barbotage d'azote pour éliminer l'oxygène et refroidie à environ 5-6 °C. 5 cm³ d'une solution à 0,42% en masse d'hydroperoxyde de cumène dans l'ISOPAR^{™}H sont ajoutés à l'émulsion maintenue sous agitation, puis une solution aqueuse à 0,1% en masse de métabisulfite de sodium est introduite progressivement à un débit de 0,5 cm³ par minute pour initier la réaction de polymérisation. La température du milieu augmente jusqu'à atteindre un palier.

Après un maintien à 80°C pendant une heure, l'Isopar^{™}H est retiré du milieu réactionnel par distillation sous vide partiel, ainsi qu'une majeure partie de l'eau. Le milieu réactionnel est ensuite refroidi jusqu'à environ 35°C pour obtenir le mélange noté (M₁).

Le mélange (M₁) précédemment obtenu est fractionné en différentes portions auxquelles sont ajoutées les différentes compositions tensioactives (EM₁), (EM₂) et (EM₃), telles que décrites ci-dessus, préalablement chauffées à 60 °C, dans des proportions massiques telles qu'indiquées dans le tableau 2 ci-dessous.

Les latex inverses auto-inversibles résultant de ces mélanges sont respectivement notés (LI₁), (LI₂), et (LI₃) sont évalués par l'observation de leur aspect à 25°C, par la vitesse d'inversion lors de la préparation d'un gel aqueux à 2% massique de latex inverse auto-inversible (dont la méthode est décrite ci-dessous), par la viscosité de ce gel aqueux à 2% massique d'un latex inverse auto-inversible.

La méthode d'évaluation de la durée d'inversion des latex inverses auto-inversibles consiste à introduire dans un bécher de 2 litres, la quantité d'eau nécessaire à la préparation d'un gel aqueux de 800 grammes. On place vers le fond bécher une hélice d'agitateur mécanique de type Turbotest^{™} version 2004 commercialisé par la société VMI, connectée à un moteur. L'agitation est démarrée à une vitesse de 900 tours /minute et la quantité nécessaire de latex inverse auto-inversible à évaluer est introduite dans le bécher sous agitation. L'agitation crée un vortex qui disparaît lorsque le polymère s'inverse et le gel se forme. La durée d'inversion, mesurée en secondes, des latex inverses auto-inversibles correspond au temps écoulé entre le début de l'ajout du latex inverse auto-inversible testé et la disparition du vortex, conduisant à l'obtention d'un gel lisse, exempt de grumeaux. Cette évaluation est réalisée à l'issue de la fabrication des latex inverses testés (t = 0), puis après une période de stockage de 2 mois à 25°C (t = 2 mois). Les résultats obtenus sont consignés dans le tableau 2 ci-dessous. La viscosité d'un gel aqueux à 2% massique de latex inverse auto-inversible (µ), est mesurée à t = 0 puis à t = 2 mois, au moyen d'un viscosimètre Brookfield RVT (Mobile 6 Vitesse 5). De même, l'aspect du latex inverse auto- inversible est évaluée visuellement à t = 0.

**Tableau 2**

| | Latex inverses auto-inversibles | | |
|---|---|---|---|
| | (LI₁) | (LI₂) | (LI₃) |

| | Référence de la composition tensioactive testée | | |
|---|---|---|---|
| | (EM₁) | (EM₂) | (EM₃) |
| Quantité testée (EMi)/(Lli) (% massique) | 5% | 7% | 7% |

| | Mesures à t = 0 | | |
|---|---|---|---|
| µ (en mPas) | 91.000 | 90.000 | 75.000 |
| Durée d'inversion | 82s | 22s | 14s |
| Aspect du latex auto-inversible à 25°C | EII* | EII* | EII* |

| | Mesures à t = 2 mois | | |
|---|---|---|---|
| µ (en mPas) | 62.000 | 65.000 | 77.000 |
| Durée d'inversion | 142s | 22s | 12s |

### EII* : Emulsion laiteuse liquide

Les latex inverses auto-inversibles (LI₂) et (LI₃) selon l'invention, et exempts de dérivés alkoxylés, permettent d'obtenir des gels lisses, avec une durée d'inversion largement inférieure à celle observée pour le latex inverse auto-inversible (LI₁), ne comprenant que le seul monolaurate de décaglycérol comme constituant du système tensioactif inverseur, tout en conservant des propriétés épaississantes excellentes. De plus, ils se caractérisent par une meilleure reproductibilité de la vitesse d'inversion et des propriétés épaississantes après deux mois de stockage que pour le latex inverse auto-inversible comparatif (LI₁).

### III- Préparation et évaluation de latex inverses auto-inversibles d'un copolymère réticulé du sel de sodium de l'acide 2-méthyl-[(1-oxo-2-propènyl) amino] 1-propanesulfonique et de l'acrylamide.

On prépare une phase aqueuse en versant successivement dans un bécher et sous agitation 255 grammes d'une solution commerciale à 50% massique d'acrylamide, 272 grammes d'une solution aqueuse à 55% de sel de sodium d'acide 2-méthyl-[(1-oxo-2-propenyl) amino] 1-propanesulfonique, 0,107 gramme de méthylène bis(acrylamide), 0,45 gramme d'une solution aqueuse commerciale à 40% en masse de diéthylènetriamine penta-acétate de sodium, et 45 grammes d'une solution aqueuse à 48% en masse d'hydroxyde de sodium pour ajuster le pH de cette phase aqueuse à 6,0.

On prépare indépendamment une phase organique en mélangeant 220 grammes d'Emogreen^{™}L15 et 25 grammes de Simaline^{™}IE.

La phase aqueuse préparée est ensuite ajoutée progressivement sur la phase huileuse puis dispersée à l'aide d'un rotor stator type Ultra Turrax commercialisé par la société Ika.

L'émulsion obtenue est transférée dans un réacteur pour être soumise à un barbotage d'azote pour éliminer l'oxygène et refroidie à environ 5-6 °C. 0,69 gramme de persulfate de sodium préalablement dissout dans 2 grammes d'eau est ajouté à l'émulsion maintenue sous agitation, puis une solution aqueuse à 2,25% n masse de métabisulfite de sodium est introduite progressivement pour initier la réaction de polymérisation. La température du milieu augmente jusqu'à atteindre un palier.

Après un maintien à 80°C pendant trois heures, le milieu réactionnel est ensuite refroidi jusqu'à environ 35°C pour obtenir le mélange noté (M₂).

Le mélange (M₂) précédemment obtenu est fractionné en différentes portions auxquelles sont ajoutées les différentes compositions tensioactives (EM₁), (EM₂) et (EM₃), telles que décrites ci-dessus, préalablement chauffées à 60 °C, dans des proportions massiques telles qu'indiquées dans le tableau 3 ci-dessous.

Les latex inverses auto-inversibles résultant de ces mélanges sont respectivement notés (LI₄), (LI₅) et (LI₆) sont évalués par l'observation de leur aspect à 25°C, par la vitesse d'inversion lors de la préparation d'un gel aqueux à 2% massique de latex inverse auto-inversible (dont la méthode est décrite ci-dessous), par la viscosité de ce gel aqueux à 2% massique d'un latex inverse auto-inversible.

La méthode d'évaluation de la durée d'inversion des latex inverses auto-inversibles consiste à introduire dans un bécher de 2 litres, la quantité d'eau nécessaire à la préparation d'un gel aqueux de 800 grammes. On place vers le fond bécher une hélice d'agitateur mécanique de type Turbotest^{™} version 2004 commercialisé par la société VMI, connectée à un moteur. L'agitation est démarrée à une vitesse de 900 tours /minute et la quantité nécessaire de latex inverse auto-inversible à évaluer est introduite dans le bécher sous agitation. L'agitation crée un vortex qui disparaît lorsque le polymère s'inverse et le gel se forme. La durée d'inversion, mesurée en secondes, des latex inverses auto-inversibles correspond au temps écoulé entre le début de l'ajout du latex inverse auto-inversible testé et la disparition du vortex, conduisant à l'obtention d'un gel lisse, exempt de grumeaux. Cette évaluation est réalisée à l'issue de la fabrication des latex inverses testés (t = 0). Les résultats obtenus sont consignés dans le tableau 3 ci-dessous. La viscosité d'un gel aqueux à 2% massique de latex inverse auto-inversible (µ), est mesurée à t = 0 au moyen d'un viscosimètre Brookfield RVT (Mobile 6 Vitesse 5). De même, l'aspect du latex inverse auto- inversible est évaluée visuellement à t = 0.

**Tableau 2**

| | Latex inverses auto-inversibles | | |
|---|---|---|---|
| | (LI₄) | (LI₅) | (LI₆) |

| | Référence de la composition tensioactive testée | | |
|---|---|---|---|
| | (EM₁) | (EM₂) | (EM₃) |
| Quantité testée (EMi)/(Lli) (% massique) | 5% | 7% | 7% |

| | Mesures à t = 0 | | |
|---|---|---|---|
| µ (en mPas) | 75.000 | 101.200 | 83.600 |
| Durée d'inversion | 45s | 30s | 30s |
| Aspect du latex auto-inversible à 25°C | Ell* | Ell* | Ell* |

### Ell* : Emulsion laiteuse liquide

Les latex inverses auto-inversibles (LI₅) et (LI₆) selon l'invention, et exempts de dérivés alkoxylés, permettent d'obtenir à t = 0, des gels lisses, avec une durée d'inversion inférieure à celle observée pour le latex inverse auto-inversible (LI₁), ne comprenant que le seul monolaurate de décaglycérol comme constituant du système tensioactif inverseur, tout en conservant des propriétés épaississantes excellentes.

### IV : Formulations détergentes illustratives

Dans les formulations suivantes, les pourcentages sont exprimés en pourcentage massique pour 100% de la masse de la formulation.

### IV_{A} - Composition nettoyante pour fours et grilles de cuissons

| Ingrédients | Teneur massique |
|---|---|
| SIMULSOL^{™}OX1309L⁽¹⁾ | 2% |
| SIMULSOL^{™}SL7G⁽²⁾ | 2% |
| Composition (LI₂) | 6% |
| Hydroxyde de sodium : | 25% |
| Eau : | qs 100% |

| | |
|---|---|
| (1) : SIMULSOL^{™} OX1309L : composition tensioactive détergente commercialisée par la société SEPPIC, comprenant des alcools polyéthoxylés résultant de la réaction d'un équivalent molaire d'un alcool commercialisé sous le nom de marque EXXAL^{™}13 avec 9 équivalents molaires d'oxyde d'éthylène. (2) : SIMULSOL^{™}SL7G : solution de n-heptyl polyglucosides, agent hydrotrope et solubilisant commercialisé par la société SEPPIC. | |

### Préparation

a) Un pré-gel est préparé à 20°C par l'addition du SIMULSOL^{™}OX1309L, puis de le SIMULSOL^{™}SL7G dans l'eau. La composition (LI₂) selon l'invention est ensuite 10 introduite dans la solution aqueuse et mélangé jusqu'à l'obtention d'un gel de viscosité stable.
b) La soude est ensuite introduit progressivement sous agitation mécanique à une température de 20°C jusqu'à l'obtention d'un gel homogène. Le gel obtenu à l'issue de l'étape b) est d'un aspect homogène et limpide, de 15 viscosité 10.000 mPa.s (Brookfield LVT à une vitesse de 6 tours/minute). Après une période de stockage de 6 mois à 25°C, le gel obtenu à l'issue de l'étape b) de ce mode opératoire présente un aspect homogène et limpide, de viscosité 12.000 mPa.s (Brookfield LVT, à une vitesse de 6 tours/minute).

### Procédé de nettoyage

La composition préparée ci-dessus est pulvérisée à température ambiante sur les parois d'un four souillé par des graisses alimentaires et sur les grilles de cuissons également souillées par des graisses alimentaires. Après dix minutes, les parois du four et les grilles de cuisson sont rincées à l'aide d'eau chaude à 60°C. Les parois du four et la surfaces des grilles de cuisson ainsi nettoyé ne présentent plus de salissures.

### IV_{B} - Nettoyant pour surfaces en aluminium

| Ingrédients | Teneur massique |
|---|---|
| SIMULSOL^{™}OX1309L | 3% |
| SIMULSOL^{™}SL7G | 3% |
| Composition (LI₂) | 5% |
| Acide phosphorique à 75% | 40% |
| HORDAPHOS⁽³⁾ MDGB 1% | 5% |
| Dipropylèneglycol méthyl éther | 5% |
| Eau : | qs 100% |

| | |
|---|---|
| (3) : HORDAPHOS^{™} MDGB est une composition à base d'esters phosphoriques, utilisée comme agent anti-corrosion. | |

### Préparation

Chaque ingrédient est introduit successivement dans une cuve de mélange sous agitation mécanique modérée, à température ambiante, jusqu'à l'obtention d'une composition homogène et limpide. L'agitation est maintenue pendant 30 minutes à 20°C. La composition obtenue présente une valeur de pH mesurée inférieure à 1,0 et est limpide et homogène après stockage pendant une durée d'un mois à 40°C.

### Procédé de nettoyage

On dilue à 3% dans l'eau la composition préparée au paragraphe précédent et la solution ainsi obtenue est pulvérisée sur la paroi en aluminium à nettoyer. Cette paroi est ensuite rincée avec de l'eau chaude à 60°C.

## Revendications

1. Composition aqueuse liquide détergente (F) à usage ménager ou industriel, **caractérisée en ce qu'**elle comprend comme agent épaississant, pour 100% de sa masse totale entre 0,1% et 10% massique un latex inverse auto-inversible d'un polyélectrolyte anionique réticulé (P) comprenant, pour 100% molaire :
**(a₁)** - d'une proportion supérieure ou égale à 25% molaire et inférieure ou égale à 80% molaire, d'unités monomériques issues de l'acide 2-méthyl 2-[(1-oxo 2-propènyl) amino] 1-propanesulfonique sous forme acide libre ou partiellement ou totalement salifiée ;
**(a₂)** - d'une proportion supérieure ou égale à 20% molaire et inférieure ou égale à 75% molaire, d'unités monomériques issues d'au moins un monomère choisi parmi les éléments du groupe constitué par l'acrylamide, le N,N-diméthyl acrylamide ; le méthacrylamide ou le N-isopropyl acrylamide ;
**(a₃)** - optionnellement d'une proportion supérieure à 0% molaire et inférieure ou égale à 10% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée ;
**(a₄)** - d'une proportion supérieure à 0% molaire et inférieure ou égale à 1% molaire d'unités monomériques issues d'au moins un monomère de réticulation (AR) diéthylénique ou polyéthylénique;
la somme des dites proportions molaires en unités monomériques selon a₁), a₂) a₃) et a₄) étant égale à 100% molaire;
ledit latex inverse auto-inversible étant une émulsion de type eau-dans-huile (E) comprenant pour 100% de sa masse :
**a)**- de 10% massique à 90% massique, dudit polyélectrolyte anionique réticulé (P) ;
**b)**- De 5% massique à 50% massique, d'une phase grasse constituée d'au moins une huile (H),
**c)** - De 1% massique à 50% massique d'eau,
**d)** - De 0,5% massique à 10% massique d'un système émulsionnant de type eau-dans-huile (S₁), et
**e)**- De 2% massique à 10% massique d'un système émulsionnant de type huile-dans-eau (S₂) ;
la somme des proportions massiques en composés selon a), b), c), d) et e) étant égale à 100% massique ;
ledit système émulsionnant de type huile-dans-eau (S₂) du latex inverse auto-inversible comprenant pour 100% de sa masse :
**f)** - Une proportion supérieure ou égale à 50% massique et inférieure ou égale à 100% d'une composition (Ce) qui comprend pour 100% de sa masse :
**e₁)** - De 10% massique à 60% massique d'au moins un composé de formule (I) :
HO-[CH₂-CH(OH)-CH₂-O]ₙ-H (I)
dans laquelle n représente un nombre entier supérieur ou égal à un et inférieur ou égal à quinze ;
**e₂) -** De 40% massique à 90 % massique d'au moins un composé de formule (II) :
R₁-(C=O)-[O-CH₂-CH(OH)-CH₂]ₚ-OH (II),
dans laquelle p, différent ou identique à n, représente un nombre entier supérieur ou égal à un et inférieur ou égal à quinze ; et dans laquelle le groupe R₁-(C=O)- représente un radical aliphatique saturé ou insaturé, linéaire ou ramifié, comportant de six à vingt-deux atomes de carbone, et optionnellement
**e₃)** - Jusqu'à 30% massique d'au moins une composition (C₁₁) représentée par la formule (III) :
HO-[CH₂-CHOH-CH₂-O-]_{q}-(G)ᵣ-H (III),
dans laquelle q différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00,
ladite composition (C₁₁) consistant en un mélange des composés de formules (III₁), (III₂), (III₃), (III₄) et (III₅) :
HO-[CH₂-CHOH-CH₂-O-]_{q} -O-(G)₁-H (III₁),
HO-[CH₂-CHOH-CH₂-O-]_{q} -O-(G)₂-H (III₂),
HO-[CH₂-CHOH-CH₂-O-]_{q} -O-(G)₃-H (III₃),
HO-[CH₂-CHOH-CH₂-O-]_{q} -O-(G)₄-H (III₄),
HO-[CH₂-CHOH-CH₂-O-]_{q} -O-(G)₅-H (III₅),
en des proportions molaires en dits composés de formules (III₁), (III₂), (III₃), (III₄) et (III₅) respectivement égales à a₁, a₂, a₃, a₄ et a₅, telles que la somme (a₁ + a₂ + a₃ + a₄ + a₅) est égale à un, et que la somme (a₁ + 2a₂ + 3a₃ + 4a₄ + 5a₅) est égale à r ;
la somme des proportions massiques en composés selon e₁), e₂) et e₃) étant égale à 100% massique.

2. Composition aqueuse liquide détergente (F) à usage ménager ou industriel telle que définie à la revendication 1, **caractérisée en ce que** le polyélectrolyte anionique réticulé (P) du latex inverse auto-inversible est un copolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium et de l'acrylamide ; un copolymère réticulé par le triallylamine et/ou le méthylène-bis(acrylamide), de l'acide 2-méthyl 2-[(1-oxo 2-propènyl) amino] 1-propanesulfonique (γ) partiellement ou totalement salifié sous forme de sel de sodium, et de l'acrylamide (ε), dans un rapport molaire (γ)/(ε) supérieur ou égal à 30/70 et inférieur ou égal à 90/10 ; un copolymère réticulé par le triallylamine et/ou le méthylène-bis(acrylamide), de l'acide 2-méthyl 2-[(1-oxo 2-propènyl) amino] 1-propanesulfonique (γ) partiellement ou totalement salifié sous forme de sel de sodium, et d'acrylamide, (ε) dans un rapport molaire (γ)/(ε) supérieur ou égal à 40/60 et inférieur ou égal à 90/10 ; un terpolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium, de l'acrylamide et de l'acide acrylique partiellement ou totalement salifiés sous forme de sel de sodium ou de sel d'ammonium ; un terpolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 45% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 10%, ou un terpolymère réticulé par le triallylamine et/ou le méthylène-bis(acrylamide), de l'acide 2-méthyl 2-[(1-oxo 2-propènyl) amino] 1-propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 47% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 8%.

3. Composition aqueuse liquide détergente (F) à usage ménager ou industriel telle que définie à l'une quelconque des revendications 1 ou 2, **caractérisée en ce que** dans la formule (I) telle que définie précédemment, n représente un nombre entier supérieur ou égal à un et inférieur ou égal à dix, **et en ce que** dans la formule (II) telle que définie précédemment, p, identique ou différent de n, représente un nombre entier supérieur ou égal à un et inférieur ou égal à dix, et le groupe R₁-(C=O)- est choisi parmi les radicaux octanoyle, décanoyle, ω-undécylènoyle, dodécanoyle, tétradécanoyle, hexadécanoyle, octadécanoyle, 9-octadécènoyle ou 9,12-octadécadiènoyle.

4. Composition aqueuse liquide détergente (F) à usage ménager ou industriel telle que définie à l'une quelconque des revendications 1 à 3, **caractérisée en ce que** dans ledit système émulsionnant de type huile-dans-eau (S₂) du latex inverse auto-inversible, la composition (Cₑ) telle que définie précédemment, consiste en, pour 100% de sa masse :
**e₁)** - De 10% massique à 60% massique d'au moins un composé de formule (I) telle que définie précédemment et
**e₂)** - De 40% massique à 90 % massique d'au moins un composé de formule (II) telle que définie précédemment.

5. Composition aqueuse liquide détergente (F) à usage ménager ou industriel telle que définie à l'une quelconque des revendications 1 à 3, **caractérisée en ce que** dans la formule (III) telle que définie précédemment, q est égal à un, G représente le reste du glucose et r représente un nombre décimal supérieur ou égal à 1,05 et inférieur ou égal à 2,5.

6. Composition aqueuse liquide détergente (F) à usage ménager ou industriel telle que défini à l'une quelconque des revendications 1 à 3 ou 5, **caractérisée en ce que** dans ledit système émulsionnant de type huile-dans-eau (S₂) du latex inverse auto-inversible, ladite composition (Cₑ) telle que définie précédemment consiste en, pour 100% de sa masse :
**e₁)** - De 5% massique à 15% massique d'au moins un composé de formule (I) telle que définie précédemment,
**e₂)** - De 60% massique à 80 % massique d'au moins un composé de formule (II) telle que définie précédemment, et
**e₃)** - De 5% à 15% massique d'au moins une composition (C₁₁) représentée par la formule (III) telle que définie précédemment.

7. Composition aqueuse liquide détergente (F) à usage ménager ou industriel telle que définie à l'une quelconque des revendications 1 à 6, **caractérisée en ce que** ledit système émulsionnant de type huile-dans-eau (S₂) du latex inverse auto-inversible est ladite composition (Cₑ) telle que définie précédemment.

8. Utilisation comme agent épaississant et/ou émulsionnant et/ou stabilisant d'une composition aqueuse liquide détergente à usage ménager ou industriel d'un latex inverse auto-inversible telle que défini dans l'une quelconque des revendications 1 à 7.

9. Utilisation de ladite composition aqueuse liquide détergente (F) telle que définie à la revendication 8, pour nettoyer les surfaces dures.

10. Procédé de nettoyage d'une surface dure, **caractérisé en ce qu'**il comprend au moins une étape a"1) d'application de la composition aqueuse liquide détergente (F) telle que définie à l'une quelconque des revendications 1 à 7, suivie d'au moins une étape b"1) de rinçage de ladite surface dure.

## Patentansprüche

1. Wässrige flüssige Reinigungsmittelzusammensetzung (F) für den Gebrauch im Haushalt oder in der Industrie, **dadurch gekennzeichnet, dass** sie als Verdickungsmittel pro 100 % ihrer Gesamtmasse zwischen 0,1 Massen-% und 10 Massen-% eines selbstinvertierenden inversen Latex eines vernetzten anionischen Polyelektrolyten (P) umfasst, der pro 100 Mol-% Folgendes umfasst:
(a₁) - einen Anteil von mehr als oder gleich 25 Mol-% und weniger als oder gleich 80 Mol-% an Monomereinheiten, die von 2-Methyl-2-[(1-oxo-2-propenyl)amino]-1-propansulfonsäure, in Form der freien Säure oder partiell oder vollständig versalzt, stammen,
(a₂) - einen Anteil von mehr als oder gleich 20 Mol-% und weniger als oder gleich 75 Mol-% an Monomereinheiten, die von mindestens einem Monomer stammen, das aus den Elementen der aus Acrylamid, N,N-Dimethylacrylamid, Methacrylamid oder N-Isopropylacrylamid bestehenden Gruppe ausgewählt ist,
(a₃) - optional einen Anteil von mehr als 0 Mol-% und weniger als oder gleich 10 Mol-% an Monomereinheiten, die von mindestens einem Monomer stammen, das aus den Elementen der aus Acrylsäure, Methacrylsäure, 2-Carboxyethylacrylsäure, Itaconsäure, Maleinsäure, 3-Methyl-3-[(1-oxo-2-propenyl)amino]butansäure bestehenden Gruppe ausgewählt ist, wobei die Carboxylfunktion der Monomere in Form der freien Säure, partiell versalzt oder vollständig versalzt ist, und
(a₄) - einen Anteil von mehr als 0 Mol-% und weniger als oder gleich 1 Mol-% an Monomereinheiten, die von mindestens einem diethylenisch oder polyethylenisch vernetzenden Monomer (AR) stammen,
wobei die Summe der molaren Anteile der Monomereinheiten gemäß a₁), a₂) a₃) und a₄) gleich 100 Mol-% ist,
wobei der selbstinvertierende inverse Latex eine Emulsion des Wasser-in-Öl-Typs (E) ist, die pro 100 % ihrer Masse Folgendes umfasst:
a) - von 10 Massen-% bis 90 Massen-% des vernetzten anionischen Polyelektrolyten (P),
b) - von 5 Massen-% bis 50 Massen-% einer aus mindestens einem Öl (H) bestehenden Fettphase,
c) - von 1 Massen-% bis 50 Massen-% Wasser,
d) - von 0,5 Massen-% bis 10 Massen-% eines Emulgiersystems des Wasser-in-Öl-Typs (S₁) und
e) - von 2 Massen-% bis 10 Massen-% eines Emulgiersystems des Öl-in-Wasser-Typs (S₂),
wobei die Summe der Massenanteile der Verbindungen gemäß a), b), c), d) und e) gleich 100 Massen-% ist,
wobei das Emulgiersystem des Öl-in-Wasser-Typs (S₂) des selbstinvertierenden inversen Latex pro 100 % seiner Masse Folgendes umfasst:
f) - einen Anteil von mehr als oder gleich 50 Massen-% und weniger als oder gleich 100 % einer Zusammensetzung (Ce), die pro 100 % ihrer Masse Folgendes umfasst:
e₁) - von 10 Massen-% bis 60 Massen-% mindestens einer Verbindung der Formel (I):
HO-[CH₂-CH(OH)-CH₂-O]ₙ-H (I),
worin n eine ganze Zahl größer als oder gleich eins und kleiner als oder gleich fünfzehn darstellt,
e₂) - von 40 Massen-% bis 90 Massen-% mindestens einer Verbindung der Formel (II):
R₁-(C=O)-[O-CH₂-CH(OH)-CH₂]ₚ-OH (II),
worin p, das verschieden von oder gleich n ist, eine ganze Zahl größer als oder gleich eins und kleiner als oder gleich fünfzehn darstellt und worin die Gruppe R₁-(C=O)- einen geraden oder verzweigten, gesättigten oder ungesättigten aliphatischen Rest mit sechs bis zweiundzwanzig Kohlenstoffatomen darstellt, und optional e₃) - bis zu 30 Massen-% mindestens einer Zusammensetzung (C₁₁), dargestellt durch die Formel (III):
HO-[CH₂-CHOH-CH₂-O-]_{q}-(G)ᵣ-H (III),
worin q, das verschieden von oder gleich n ist, eine ganze Zahl größer als oder gleich eins und kleiner als oder gleich 3 darstellt, G den Rest eines reduzierenden Zuckers darstellt und r eine Dezimalzahl größer als oder gleich 1,05 und kleiner als oder gleich 5,00 darstellt, wobei die Zusammensetzung (C₁₁) aus einem Gemisch der Verbindungen der Formeln (III₁), (III₂), (III₃), (III₄) und (III₅):
HO-[CH₂-CHOH-CH₂-O-]_{q} -O-(G)₁-H (III₁),
HO-[CH₂-CHOH-CH₂-O-]_{q} -O-(G)₂-H (III2),
HO-[CH₂-CHOH-CH₂-O-]_{q} -O-(G)₃-H (III₃),
HO-[CH₂-CHOH-CH₂-O-]_{q} -O-(G)₄-H (III₄),
HO-[CH₂-CHOH-CH₂-O-]_{q} -O-(G)₅-H (III.₅)
in molaren Anteilen dieser Verbindungen der Formeln (III₁), (III₂), (III₃), (III₄) und (III₅) besteht, die gleich a₁, a₂, a₃, a₄ bzw. a₅ sind, so dass die Summe (a₁ + a₂ + a₃ + a₄ + a₅) gleich eins ist und die Summe (a₁ + 2a₂ + 3a₃ + 4a₄ + 5a₅) gleich r ist,
wobei die Summe der Massenanteile der Verbindungen gemäß e₁), e₂) und e₃) gleich 100 Massen-% ist.

2. Wässrige flüssige Reinigungsmittelzusammensetzung (F) für den Gebrauch im Haushalt oder in der Industrie nach Anspruch 1, **dadurch gekennzeichnet, dass** der vernetzte anionische Polyelektrolyt (P) des selbstinvertierenden inversen Latex ein durch Triallylamin und/oder Methylenbis(acrylamid) vernetztes Copolymer von partiell oder vollständig in Form des Natriumsalzes oder des Ammoniumsalzes versalzter 2-Methyl-2-[(1-oxo-2-propenyl)amino]-1-propansulfonsäure und Acrylamid ist, ein durch Triallylamin und/oder Methylenbis(acrylamid) vernetztes Copolymer von partiell oder vollständig in Form des Natriumsalzes versalzter 2-Methyl-2-[(1-oxo-2-propenyl)amino]-1-propansulfonsäure (γ) und Acrylamid (ε) in einem molaren Verhältnis (γ)/(ε) größer als oder gleich 30/70 und kleiner als oder gleich 90/10 ist, ein durch Triallylamin und/oder Methylenbis(acrylamid) vernetztes Copolymer von partiell oder vollständig in Form des Natriumsalzes versalzter 2-Methyl-2-[(1-oxo-2-propenyl)amino]-1-propansulfonsäure (γ) und Acrylamid (ε) in einem molaren Verhältnis (γ)/(ε) größer als oder gleich 40/60 und kleiner als oder gleich 90/10 ist, ein durch Triallylamin und/oder Methylenbis(acrylamid) vernetztes Terpolymer von partiell oder vollständig in Form des Natriumsalzes oder des Ammoniumsalzes versalzter 2-Methyl-2-[(1-oxo-2-propenyl)amino]-1-propansulfonsäure, Acrylamid und partiell oder vollständig in Form des Natriumsalzes oder des Ammoniumsalzes versalzter Acrylsäure ist, ein durch Triallylamin und/oder Methylenbis(acrylamid) vernetztes Terpolymer von partiell oder vollständig in Form des Natriumsalzes oder des Ammoniumsalzes versalzter 2-Methyl-2-[(1-oxo-2-propenyl)amino]-1-propansulfonsäure in einem molaren Anteil von mehr als oder gleich 30 % und weniger als oder gleich 45 %, Acrylamid in einem molaren Anteil von mehr als oder gleich 45 % und weniger als oder gleich 68 % und partiell oder vollständig in Form des Natriumsalzes oder des Ammoniumsalzes versalzter Acrylsäure in einem molaren Anteil von mehr als oder gleich 2 % und weniger als oder gleich 10 % oder ein durch Triallylamin und/oder Methylenbis(acrylamid) vernetztes Terpolymer von partiell oder vollständig in Form des Natriumsalzes oder des Ammoniumsalzes versalzter 2-Methyl-2-[(1-oxo-2-propenyl)amino]-1-propansulfonsäure in einem molaren Anteil von mehr als oder gleich 30 % und weniger als oder gleich 45 %, Acrylamid in einem molaren Anteil von mehr als oder gleich 47 % und weniger als oder gleich 68 % und partiell oder vollständig in Form des Natriumsalzes oder des Ammoniumsalzes versalzter Acrylsäure in einem molaren Anteil von mehr als oder gleich 2 % und weniger als oder gleich 8 % ist.

3. Wässrige flüssige Reinigungsmittelzusammensetzung (F) für den Gebrauch im Haushalt oder in der Industrie nach einem der Ansprüche 1 oder 2, **dadurch gekennzeichnet, dass** in der vorstehenden Formel (I) n eine ganze Zahl größer als oder gleich eins und kleiner als oder gleich zehn darstellt, und dadurch, dass in der vorstehenden Formel (II) p, das gleich oder verschieden von n ist, eine ganze Zahl größer als oder gleich eins und kleiner als oder gleich zehn darstellt und die Gruppe R₁-(C=O)- aus Octanoyl-, Decanoyl-, w-Undecylenoyl-, Dodecanoyl, Tetradecanoyl, Hexadecanoyl, Octadecanoyl, 9-Octadecenoyl oder 9,12-Octadecenoyl-Resten ausgewählt ist.

4. Wässrige flüssige Reinigungsmittelzusammensetzung (F) für den Gebrauch im Haushalt oder in der Industrie nach einem der Ansprüche 1 bis 3, **dadurch gekennzeichnet, dass** in dem Emulgiersystem des Öl-in-Wasser-Typs (S₂) des selbstinvertierenden inversen Latex die vorstehende Zusammensetzung (Cₑ) pro 100 % ihrer Masse aus Folgendem besteht:
e₁) - von 10 Massen-% bis 60 Massen-% mindestens einer vorstehenden Verbindung der Formel (I) und
e₂) - von 40 Massen-% bis 90 Massen-% von mindestens einer vorstehenden Verbindung der Formel (II).

5. Wässrige flüssige Reinigungsmittelzusammensetzung (F) für den Gebrauch im Haushalt oder in der Industrie nach einem der Ansprüche 1 bis 3, **dadurch gekennzeichnet, dass** in der vorstehenden Formel (III) q gleich eins ist, G den Rest von Glucose darstellt und r eine Dezimalzahl größer als oder gleich 1,05 und kleiner als oder gleich 2,5 darstellt.

6. Wässrige flüssige Reinigungsmittelzusammensetzung (F) für den Gebrauch im Haushalt oder in der Industrie nach einem der Ansprüche 1 bis 3 oder 5, **dadurch gekennzeichnet, dass** in dem Emulgiersystem des Öl-in-Wasser-Typs (S₂) des selbstinvertierenden inversen Latex die vorstehende Zusammensetzung (Cₑ) pro 100 % ihrer Masse aus Folgendem besteht:
e₁) - von 5 Massen-% bis 15 Massen-% mindestens einer vorstehenden Verbindung der Formel (I), e₂) - von 60 Massen-% bis 80 Massen-% mindestens einer vorstehenden Verbindung der Formel (II) und
e₃) - von 5 Massen-% bis 15 Massen-% mindestens einer durch die vorstehende Formel (III) dargestellten Zusammensetzung (C₁₁).

7. Wässrige flüssige Reinigungsmittelzusammensetzung (F) für den Gebrauch im Haushalt oder in der Industrie nach einem der Ansprüche 1 bis 6, **dadurch gekennzeichnet, dass** das Emulgiersystem des Öl-in-Wasser-Typs (S₂) des selbstinvertierenden inversen Latex die vorstehende Zusammensetzung (Cₑ) ist.

8. Verwendung einer wässrigen flüssigen Reinigungsmittelzusammensetzung für den Gebrauch im Haushalt oder in der Industrie eines selbstinvertierenden inversen Latex nach einem der Ansprüche 1 bis 7 als Verdickungs- und/oder Emulgier- und/oder Stabilisierungsmittel.

9. Verwendung der wässrigen flüssigen Reinigungsmittelzusammensetzung (F) nach Anspruch 8 zum Reinigen harter Oberflächen.

10. Verfahren zur Reinigung einer harten Oberfläche, **dadurch gekennzeichnet, dass** es mindestens einen Schritt a"1) des Aufbringens der wässrigen flüssigen Reinigungsmittelzusammensetzung (F) nach einem der Ansprüche 1 bis 7, gefolgt von mindestens einem Schritt b"1) des Spülens der harten Oberfläche, umfasst.

## Claims

1. Aqueous liquid detergent composition (F) for household or industrial use, **characterized in that** it comprises as thickener, per 100% of its total weight, between 0.1% and 10% by weight of a self-invertible inverse latex of a crosslinked anionic polyelectrolyte (P) comprising, per 100 mol%:
(a₁) - a proportion of greater than or equal to 25 mol% and less than or equal to 80 mol% of monomer units derived from 2-methyl-2-[(1-oxo-2-propenyl)amino]-1-propanesulfonic acid in the form of the free acid or partly or entirely in the form of a salt;
(a₂) - a proportion of greater than or equal to 20 mol% and less than or equal to 75 mol% of monomer units derived from at least one monomer selected from the members of the group consisting of acrylamide, N,N-dimethylacrylamide; methacrylamide or N-isopropylacrylamide;
(a₃) - optionally, a proportion of greater than 0 mol% and less than or equal to 10 mol% of monomer units derived from at least one monomer selected from the members of the group consisting of acrylic acid, methacrylic acid, 2-carboxyethylacrylic acid, itaconic acid, maleic acid and 3-methyl-3-[(1-oxo-2-propenyl)amino]butanoic acid, the carboxylic function of said monomers being in the form of the free acid or partly or entirely in the form of a salt;
(a₄) - a proportion of greater than 0 mol% and less than or equal to 1 mol%, of monomer units derived from at least one diethylenic or polyethylenic crosslinking monomer (AR);
the sum of said molar proportions of monomer units according to a₁), a₂), a₃) and a₄) being equal to 100 mol%; said self-invertible inverse latex being a water-in-oil type emulsion (E) comprising, per 100% of its weight:
a) - from 10% by weight to 90% by weight of said crosslinked anionic polyelectrolyte (P);
b) - from 5% by weight to 50% by weight of a fatty phase consisting of at least one oil (H);
c) - from 1% by weight to 50% by weight of water;
d) - from 0.5% by weight to 10% by weight of a water-in-oil type emulsifying system (S₁); and
e) - from 2% by weight to 10% by weight of an oil-in-water type emulsifying system (S₂);
the sum of the proportions by weight of compounds according to a), b), c), d) and e) being equal to 100% by weight;
said oil-in-water type emulsifying system (S₂) of the self-invertible inverse latex comprising, per 100% of its weight:
f) - a proportion of greater than or equal to 50% by weight and less than or equal to 100% of a composition (Ce) comprising, per 100% of its weight:
e₁) - from 10% by weight to 60% by weight of at least one compound of formula (I):
HO-[CH₂-CH(OH)-CH₂-O]ₙ-H (I)
in which n represents an integer greater than or equal to 1 and less than or equal to 15;
e₂) - from 40% by weight to 90% by weight of at least one compound of formula (II):
R₁-(C=O)-[O-CH₂-CH(OH)-CH₂]ₚ-OH (II),
in which p, which may be different from or identical to n, represents an integer greater than or equal to 1 and less than or equal to 15; and in which the R₁-(C=O)- group represents a saturated or unsaturated, linear or branched, aliphatic radical comprising from 6 to 22 carbon atoms; and optionally
e₃) - up to 30% by weight of at least one composition (C₁₁) represented by formula (III):
HO-[CH₂-CHOH-CH₂-O-]_{q}-(G)ᵣ-H (III),
in which q, which may be different from or identical to n, represents an integer greater than or equal to 1 and less than or equal to 3, G represents a reducing sugar residue and r represents a decimal number greater than or equal to 1.05 and less than or equal to 5.00,
said composition (C₁₁) consisting of a mixture of the compounds of formulas (III₁), (III₂), (III₃), (III₄) and (III₅) :
HO-[CH₂-CHOH-CH₂-O-]_{q} -O-(G)₁-H (III₁),
HO-[CH₂-CHOH-CH₂-O-]_{q} -O-(G)₂-H (III₂),
HO-[CH₂-CHOH-CH₂-O-]_{q} -O-(G)₃-H (III₃),
HO-[CH₂-CHOH-CH₂-O-]_{q} -O-(G)₄-H (III₄),
HO-[CH₂-CHOH-CH₂-O-]_{q} -O-(G)₅-H (III₅),
in molar proportions of said compounds of formulas (III₁), (III₂), (III₃), (III₄) and (III₅) respectively equal to a₁, a₂, a₃, a₄ and a₅, such that the sum (a₁ + a₂ + a₃ + a₄ + a₅) is equal to 1, and such that the sum (a₁ + 2a₂ + 3a₃ + 4a₄ + 5a₅) is equal to r;
the sum of the proportions by weight of compounds according to e₁), e₂) and e₃) being equal to 100% by weight.

2. Aqueous liquid detergent composition (F) for household or industrial use as defined in Claim 1, **characterized in that** the crosslinked anionic polyelectrolyte (P) of the self-invertible inverse latex is a copolymer crosslinked with triallylamine and/or methylenebis(acrylamide) of 2-methyl-2-[(1-oxo-2-propenyl)amino]-1-propanesulfonic acid partly or entirely in the form of the sodium salt or ammonium salt and acrylamide, a copolymer crosslinked with triallylamine and/or methylenebis(acrylamide) of 2-methyl-2-[(1-oxo-2-propenyl)amino]-1-propanesulfonic acid (γ) partly or entirely in the form of the sodium salt and acrylamide (ε), in a molar ratio (γ)/(ε) greater than or equal to 30/70 and less than or equal to 90/10; a copolymer crosslinked with triallylamine and/or methylenebis(acrylamide) of 2-methyl-2-[(1-oxo-2-propenyl)amino]-1-propanesulfonic acid (γ) partly or entirely in the form of the sodium salt and acrylamide (ε), in a molar ratio (γ)/(ε) greater than or equal to 40/60 and less than or equal to 90/10; a terpolymer crosslinked with triallylamine and/or methylenebis(acrylamide) of 2-methyl-2-[(1-oxo-2-propenyl)amino]-1-propanesulfonic acid partly or entirely in the form of the sodium salt or ammonium salt, acrylamide and acrylic acid partly or entirely in the form of the sodium salt or ammonium salt, a terpolymer crosslinked with triallylamine and/or methylenebis(acrylamide) of 2-methyl-2-[(1-oxo-2-propenyl)amino]-1-propanesulfonic acid partly or entirely in the form of the sodium salt or ammonium salt, in a molar proportion of greater than or equal to 30% and less than or equal to 45%, acrylamide in a molar proportion of greater than or equal to 45% and less than or equal to 68%, and acrylic acid partly or entirely in the form of the sodium salt or ammonium salt, in a molar proportion of greater than or equal to 2% and less than or equal to 10%, or a terpolymer crosslinked with triallylamine and/or methylenebis(acrylamide) of 2-methyl-2-[(1-oxo-2-propenyl)amino]-1-propanesulfonic acid partly or entirely in the form of the sodium salt or ammonium salt, in a molar proportion of greater than or equal to 30% and less than or equal to 45%, acrylamide in a molar proportion of greater than or equal to 47% and less than or equal to 68%, and acrylic acid partly or entirely in the form of the sodium salt or ammonium salt, in a molar proportion of greater than or equal to 2% and less than or equal to 8%.

3. Aqueous liquid detergent composition (F) for household or industrial use as defined in either one of Claims 1 or 2, **characterized in that**, in formula (I) as defined previously, n represents an integer greater than or equal to 1 and less than or equal to 10, and **in that**, in formula (II) as defined previously, p, which may be identical to or different than n, represents an integer greater than or equal to 1 and less than or equal to 10, and the R₁-(C=O)- group is selected from octanoyl, decanoyl, ω-undecylenoyl, dodecanoyl, tetradecanoyl, hexadecanoyl, octadecanoyl, 9-octadecenoyl and 9,12-octadecadienoyl radicals.

4. Aqueous liquid detergent composition (F) for household or industrial use as defined in any one of Claims 1 to 3, **characterized in that**, in said oil-in water type emulsifying system (S₂) of the self-invertible inverse latex, the composition (Cₑ) as defined previously consists, per 100% of its weight, of:
e₁) - from 10% by weight to 60% by weight of at least one compound of formula (I) as defined previously and
e₂) - from 40% by weight to 90% by weight of at least one compound of formula (II) as defined previously.

5. Aqueous liquid detergent composition (F) for household or industrial use as defined in any one of Claims 1 to 3, **characterized in that**, in the formula (III) as defined previously, q is equal to 1, G represents a glucose residue and r represents a decimal number greater than or equal to 1.05 and less than or equal to 2.5.

6. Aqueous liquid detergent composition (F) for household or industrial use as defined in any one of Claims 1 to 3 or 5, **characterized in that**, in said oil-in water type emulsifying system (S₂) of the self-invertible inverse latex, said composition (Cₑ) as defined previously consists, per 100% of its weight, of:
e₁) - from 5% by weight to 15% by weight of at least one compound of formula (I) as defined previously,
e₂) - from 60% by weight to 80% by weight of at least one compound of formula (II) as defined previously, and
e₃) - from 5% by weight to 15% by weight of at least one composition (C₁₁) represented by formula (III) as defined previously.

7. Aqueous liquid detergent composition (F) for household or industrial use as defined in any one of Claims 1 to 6, **characterized in that** said oil-in water type emulsifying system (S₂) of the self-invertible inverse latex is said composition (Cₑ) as defined previously.

8. Use as thickener and/or emulsifier and/or stabilizer in an aqueous liquid detergent composition for household or industrial use of a self-invertible inverse latex as defined in any one of Claims 1 to 7.

9. Use of said aqueous liquid detergent composition (F) as defined in Claim 8, for cleaning hard surfaces.

10. Process for cleaning a hard surface, **characterized in that** it comprises at least one step a"1) of applying the aqueous liquid detergent composition (F) as defined in any one of Claims 1 to 7, followed by at least one step b"1) of rinsing said hard surface.
